# EUROPEAN PATENT APPLICATION

(11) **EP 4 512 406 A1**
(43) Date of publication of application: **26.02.2025**
(21) Application number: 23791912.1
(22) Date of filing: 20.04.2023
(51) Int. Cl.: A61K 31/7088, A61K 9/08, A61K 33/06, A61K 33/30, A61K 33/32, A61K 39/00, A61K 39/39, A61K 47/02, A61K 48/00, A61M 5/30, A61P 31/14, A61P 37/04, A61P 43/00

(54) **LIQUID PHARMACEUTICAL COMPOSITION**

(30) Priority: 20.04.2022 JP 2022069625
(71) Applicant: Daicel Corporation, Osaka-shi, Osaka 530-0011 (JP)
(72) Inventor: SAKAGUCHI, Naoki, Tokyo 108-8230 (JP)
(74) Representative: Grünecker Patent- und Rechtsanwälte PartG mbB
(86) International application number: PCT/JP2023/015717
(87) International publication number: WO 2023/204264

(57) **Abstract**

An object of the present disclosure is to provide at least the following. Specifically, provided is a technique in which high physiological activity is achieved when a liquid pharmaceutical composition containing a nucleic acid that exhibits the physiological activity in an injection target is injected into the injection target. The above-described problem is solved by a liquid pharmaceutical composition containing a nucleic acid and a divalent cation and having a total concentration of the divalent cation of 0.0406 mM or more and less than 81.1 mM. The liquid pharmaceutical composition is injected into an injection target by an injector configured to inject the liquid pharmaceutical composition into the injection target without using an injection needle.

## Description

### TECHNICAL FIELD

The present disclosure relates to a liquid pharmaceutical composition.

### BACKGROUND ART

In recent years, in regard to medical devices for administering a medicinal solution, interest in needleless injectors has been increasing from the viewpoint of a countermeasure against needle phobia, pursuit of usability, reduction of infectious wastes, and the like. In general, a needleless injector is known as an injector that uses compressed gas or a spring force as a driving force to push out a medicinal solution from a nozzle tip, thereby administering the medicinal solution into a living body (Non-Patent Literature 1). Further, a needleless injector that uses combustion energy of an ignition agent as ejection energy has been developed (Patent Document 1).

The needleless injector that uses combustion energy of an ignition agent as ejection energy is capable of delivering a medicinal solution to the nucleus or cytosol of a cell because the medicinal solution is instantaneously pushed out and administered. The usefulness of the needleless injector has been attracting attention also from the viewpoint of a drug delivery system (DDS), and application of the needleless injector to various drugs using a low molecular weight compound, a peptide, a protein, an antibody, or the like having an anticancer action has been studied. In particular, in the fields of vaccines and immunization, it has been reported that the needleless injector enables an increase in antibody titer or the induction of cellular immunity (Non-Patent Literature 2).

In the field of vaccines, big breakthroughs have occurred, such as development of vaccines against the novel coronavirus SARS-CoV-2. Among such vaccines, a vaccine containing mRNA as an active ingredient has been used worldwide after demonstration of high efficacy in clinical trials (Non-Patent Literature 3), and has had a great impact. The mRNA vaccine is a novel modality and is expected to be a technical singularity.

Meanwhile, a nucleic acid vaccine requires very high expression of a gene contained in the nucleic acid for achieving a sufficient antibody titer. In all nucleic acid vaccines against SARS-CoV-2 that are widely used in practice, lipid nanoparticles (LNPs) are used to enhance gene expression. However, development of new LNPs requires not only much labor but also a large production cost. In addition, there are safety concerns such as anaphylactic shock due to polyethylene glycol (PEG) used in LNPs. Accordingly, a technique for enhancing gene expression without using LNPs is desired. Also in a case where a nucleic acid containing a gene is administered to an injection target using a needleless injector, improvements have been made to achieve gene expression comparable to that achieved with LNPs.

### CITATION LIST

### PATENT DOCUMENT

Patent Document 1: JP 2012-61269 A

### NON-PATENT LITERATURE

Non-Patent Literature 1: Clin. Cosmet. Investig. Dermatol., 2018 May 1; 11: 231 to 238
Non-Patent Literature 2: AAPS PharmSciTech., 2019 Dec 9; 21(1): 19
Non-Patent Literature 3: About novel coronavirus vaccine of Pfizer Inc., Ministry of Health, Labour and Welfare [online], [searched on March 11, 2022], Internet <https://www.mhlw.go.jp/stf/seisakunitsuite/bunya/vaccine_pfizer.html>

### SUMMARY OF INVENTION

### TECHNICAL PROBLEM

An object of the present disclosure is to provide at least the following. That is, there is provided a technique capable of achieving high physiological activity when a liquid pharmaceutical composition containing a nucleic acid exhibiting physiological activity in an injection target is injected into the injection target.

### SOLUTION TO PROBLEM

The present inventor has found that in a case where a liquid pharmaceutical composition is injected into an injection target using an injector configured to inject the liquid pharmaceutical composition into the injection target without using an injection needle, the above-described problem can be solved when the liquid pharmaceutical composition contains a divalent cation, and the total concentration of the divalent cation is set in a predetermined range.

One aspect of the present disclosure is
a liquid pharmaceutical composition containing:
a nucleic acid; and a divalent cation, wherein
a total concentration of the divalent cation is 0.0406 mM or more and less than 81.1 mM, and
the liquid pharmaceutical composition is injected into an injection target using an injector configured to inject the liquid pharmaceutical composition into the injection target without using an injection needle.

In a preferred embodiment of the liquid pharmaceutical composition, the divalent cation is one or more selected from the group consisting of Mg²⁺, Ca²⁺, Mn²⁺, and Zn²⁺.

In another preferred embodiment, the injection of the liquid pharmaceutical composition into the injection target using the injector configured to inject the liquid pharmaceutical composition into the injection target without using an injection needle is injection into the injection target by jet injection.

In another preferred embodiment, the liquid pharmaceutical composition is for induction of cellular immunity.

Another aspect of the present disclosure is
an injector configured to inject a liquid pharmaceutical composition into an injection target without using an injection needle, the injector including:
a storage section containing the liquid pharmaceutical composition;
a nozzle section communicating with the storage section, the nozzle section having an ejection port for ejecting the liquid pharmaceutical composition toward the injection target; and
a pressurization section for pressurizing the liquid pharmaceutical composition contained in the storage section during an operation, thereby ejecting the liquid pharmaceutical composition from the ejection port toward the injection target, wherein
the liquid pharmaceutical composition contains a nucleic acid and a divalent cation, and a total concentration of the divalent cation is 0.0406 mM or more and less than 81.1 mM.

In a preferred embodiment of the injector, the divalent cation is one or more selected from the group consisting of Mg²⁺, Ca²⁺, Mn²⁺, and Zn²⁺.

Another aspect of the present disclosure is
a method for injecting a liquid pharmaceutical composition into an injection target, the liquid pharmaceutical composition containing a nucleic acid and a divalent cation and having a total concentration of the divalent cation of 0.0406 mM or more and less than 81.1 mM, the method including injecting the liquid pharmaceutical composition into the injection target using an injector configured to inject the liquid pharmaceutical composition into the injection target without using an injection needle.

In a preferred embodiment of the method, the divalent cation is one or more selected from the group consisting of Mg²⁺, Ca²⁺, Mn²⁺, and Zn²⁺.

### ADVANTAGEOUS EFFECTS OF INVENTION

The present disclosure can exhibit at least the following effect. Specifically, there can be exhibited an effect of providing a technique capable of achieving high physiological activity when a liquid pharmaceutical composition containing a nucleic acid exhibiting physiological activity in an injection target is injected into the injection target.

### BRIEF DESCRIPTION OF DRAWINGS

FIG. 1 is a diagram illustrating a schematic configuration of an injector according to an embodiment of the present disclosure.
FIG. 2 is a graph illustrating the results of Test Example 1 and Comparative Test Example 1-1 according to an embodiment of the present disclosure.
FIG. 3 is a graph illustrating the results of Comparative Test Example 1-2 and Comparative Test Example 1-3 according to an embodiment of the present disclosure.
FIG. 4 shows images (photographs substituted for drawings) illustrating the results of Test Example 2 and Comparative Test Example 2 according to an embodiment of the present disclosure.
FIG. 5 shows images (photographs substituted for drawings) illustrating the results of Test Example 4, Comparative Test Example 4-1, Comparative Test Example 4-2, and Comparative Test Example 4-3 according to an embodiment of the present disclosure.
FIG. 6 shows images (photographs substituted for drawings) illustrating the results of Test Example 5 and Comparative Test Example 5 according to an embodiment of the present disclosure.
FIG. 7 shows images (photographs substituted for drawings) illustrating the results of Test Example 6 and Comparative Test Example 6 according to an embodiment of the present disclosure.
FIG. 8 shows images (photographs substituted for drawings) illustrating the results of Test Example 7 and Comparative Test Example 7 according to an embodiment of the present disclosure.
FIG. 9 shows images (photographs substituted for drawings) illustrating the results of Test Example 8 and Comparative Test Example 8 according to an embodiment of the present disclosure.
FIG. 10 shows images (photographs substituted for drawings) illustrating the results of Test Example 9 and Comparative Test Example 9 according to an embodiment of the present disclosure.
FIG. 11 shows images (photographs substituted for drawings) illustrating the results of Test Example 10, Comparative Test Example 10-1, Comparative Test Example 10-2, and Comparative Test Example 10-3 according to an embodiment of the present disclosure.

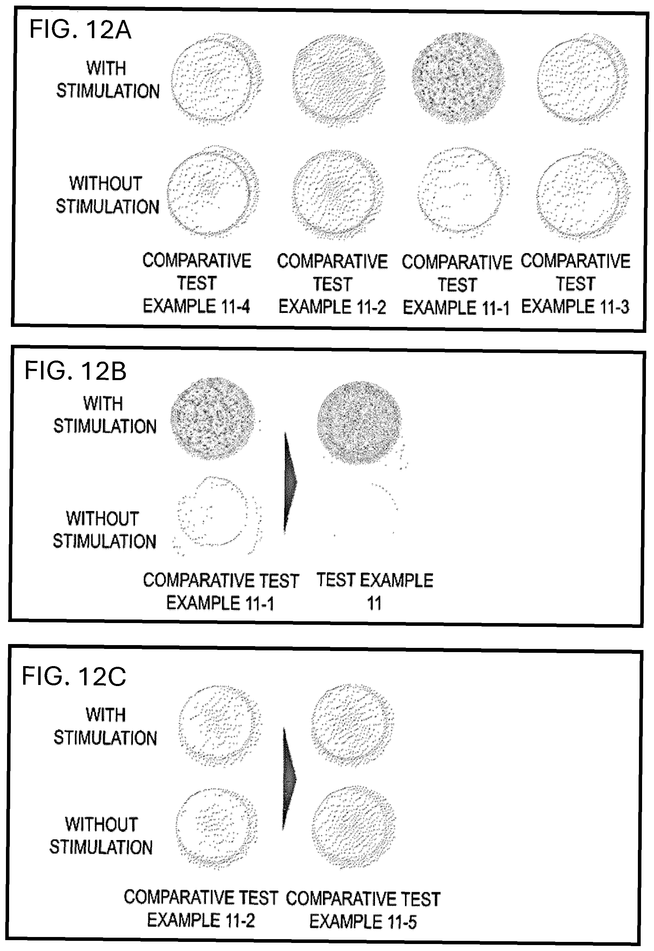

FIG. 12A shows images (photographs substituted for drawings) illustrating the results of Comparative Test Example 11-1, Comparative Test Example 11-2, Comparative Test Example 11-3, and Comparative Test Example 11-4 according to an embodiment of the present disclosure. FIG. 12B shows images (photographs substituted for drawings) illustrating the results of Test Example 11 and Comparative Test Example 11-1 according to an embodiment of the present disclosure. FIG. 12C shows images (photographs substituted for drawings) illustrating the results of Comparative Test Example 11-2 and Comparative Test Example 11-5 according to an embodiment of the present disclosure.

### DESCRIPTION OF EMBODIMENTS

The configurations, combinations thereof, and the like in the respective embodiments are examples, and various additions, omissions, substitutions, and other changes of the configurations may be made as appropriate without departing from the spirit of the present disclosure. The present disclosure is not limited by the embodiments and is limited only by the claims. Each embodiment disclosed in the present specification can be combined with any other feature disclosed herein.

One embodiment of the present disclosure is
a liquid pharmaceutical composition containing:
a nucleic acid; and a divalent cation, wherein
the total concentration of the divalent cation is 0.0406 mM or more and less than 81.1 mM, and
the liquid pharmaceutical composition is injected into an injection target using an injector configured to inject the liquid pharmaceutical composition into the injection target without using an injection needle.

### (Nucleic Acid)

The nucleic acid contained in the liquid pharmaceutical composition according to the present embodiment is not particularly limited as long as it is a nucleic acid exhibiting physiological activity in an injection target.

The nucleic acid may be DNA or RNA. The nucleic acid may be a nucleic acid including a portion encoding a protein, or a nucleic acid not including a portion encoding a protein (non-coding nucleic acid). The nucleic acid contained in the liquid pharmaceutical composition may be one type or a plurality of types.

Examples of a case where the nucleic acid is a nucleic acid including a portion encoding a protein are as follows.

For example, when the nucleic acid is DNA containing a gene, the nucleic acid exhibits physiological activity of serving as a template in synthesis (transcription) of mRNA for protein production, and the physiological activity can be quantitatively evaluated by the amount of mRNA or protein produced.

When the nucleic acid is mRNA, the nucleic acid exhibits physiological activity of serving as a template in protein synthesis (translation), and the physiological activity can be quantitatively evaluated by the amount of protein produced.

The physiological activity may be evaluated based on a change in an injection target caused due to an increase or decrease in the produced mRNA or protein. For example, in a case where the injection target is an injection target in which cellular immunity can be induced (for example, an individual (living body)), the nucleic acid is a nucleic acid encoding a protein that induces cellular immunity, and an increase in the protein that induces cellular immunity causes a change in which cellular immunity is induced in the injection target, the nucleic acid exhibits physiological activity of inducing cellular immunity, and the physiological activity can be quantitatively evaluated by the amount of cellular immunity induced in the injection target. The physiological activity may also be evaluated by the amount of cellular immunity induced in the injection target after restimulation (resensitization) with the protein that induces cellular immunity.

Examples of a case where the nucleic acid is a nucleic acid not including a portion encoding a protein are as follows.

For example, in a case where the injection target is an individual (living body) to which a vaccine can be administered, and the nucleic acid is CpG motif-containing DNA (CpG DNA) or polyinosine-polycytidylic acid (PolyI:C), the nucleic acid exhibits physiological activity as an adjuvant for enhancing a vaccine effect, and the physiological activity can be quantitatively evaluated by an antitumor effect (the effect can be evaluated by, for example, a decrease in tumor size), the degree of maturation of immune cells, or the like.

When the nucleic acid is small interfering RNA (siRNA), the nucleic acid exhibits physiological activity of degrading mRNA and suppressing expression of a target gene in a sequence-specific manner, and the physiological activity can be quantitatively evaluated by the amount of mRNA or protein produced based on the target gene.

When the nucleic acid is antisense DNA or antisense RNA, the nucleic acid exhibits physiological activity such as inhibition of splicing or inhibition of translation by hybridizing with RNA of a target gene, and the physiological activity can be quantitatively evaluated by the amount of mRNA or protein produced based on the target gene.

The nucleic acid according to the present embodiment may be in a free form or in a form fixed to a carrier such as nanoparticles, or in a modified form, and is not particularly limited, including a solvent, as long as when the nucleic acid is injected into the injection target, the nucleic acid exhibits physiological activity in the injection target, is stably present in the injection target, and does not have an adverse effect such as destruction of the injection target.

The nucleic acid may be a natural product or an artificially synthesized product.

Examples described below include an example in which free plasmid DNA containing a green fluorescence protein (GFP) gene is used as the nucleic acid and the GFP gene is used as a reporter gene, an example in which mRNA encoding GFP as a reporter protein is used, and an example in which mRNA encoding ovalbumin (OVA) is used.

In a case where the DNA contains a gene, it may be designed such that the gene is contained in an expression cassette or an expression vector. Furthermore, for example, the gene may be placed under control of a promoter suitable for the injection target and the injection site into which the DNA is to be injected. That is, known genetic engineering techniques can be used in any of the embodiments. For example, in Examples described below, CMV-DASHER-GFP (available from ATUM), which is a vector containing a GFP gene, is used as an expression vector. The plasmid vector is known and available to those skilled in the art. Subcloning of the expression vector and a recombinant vector can be performed according to a known method.

In the present embodiment, the phrase "capable of achieving high physiological activity when a liquid pharmaceutical composition containing a nucleic acid exhibiting the physiological activity in an injection target is injected into the injection target" means that in a case where the physiological activity exhibited by the nucleic acid is evaluated using a quantitative index,
the physiological activity (the physiological activity may be, for example, an amount indicating the physiological activity) exhibited by a nucleic acid when a liquid pharmaceutical composition containing the nucleic acid and a divalent cation at a total concentration outside a predetermined range described below is injected into an injection target using an injector configured to inject the liquid pharmaceutical composition into an injection target without using an injection needle is surpassed by
the physiological activity (the physiological activity may be, for example, an amount indicating the physiological activity) exhibited by a nucleic acid when a liquid pharmaceutical composition containing the nucleic acid and a divalent cation at a total concentration within the predetermined range described below is injected in the same manner as described above.

That is, for example,
in a case where the physiological activity exhibited by a nucleic acid when a liquid pharmaceutical composition containing the nucleic acid and a divalent cation at a total concentration of less than 0.0406 mM or 81.1 mM or more is injected into an injection target using an injector configured to inject the liquid pharmaceutical composition into the injection target without using an injection needle (this is referred to as embodiment B) is defined as "activity B", and
the physiological activity exhibited by a nucleic acid when a liquid pharmaceutical composition is injected into an injection target in the same manner as described above under the same conditions as in the embodiment B except that the liquid pharmaceutical composition contains the nucleic acid and the total concentration of the divalent cation is 0.0406 mM or more and less than 81.1 mM (this is referred to as embodiment A) is defined as "activity A",
activity B < activity A
is satisfied.

Alternatively, in the present embodiment, the phrase "capable of achieving high physiological activity when a liquid pharmaceutical composition containing a nucleic acid exhibiting the physiological activity in an injection target is injected into the injection target" may mean that in a case where the physiological activity exhibited by the nucleic acid is evaluated using a quantitative index,
the physiological activity (the physiological activity may be, for example, an amount indicating the physiological activity) exhibited by a nucleic acid when a liquid pharmaceutical composition containing the nucleic acid and a divalent cation at a total concentration within a predetermined range described below is injected into an injection target using a needle-equipped injector is surpassed by
the physiological activity (the physiological activity may be, for example, an amount indicating the physiological activity) exhibited by a nucleic acid when a liquid pharmaceutical composition containing the nucleic acid and a divalent cation at a total concentration within the predetermined range described below is injected into an injection target in the same manner as described above except for using an injector configured to inject the liquid pharmaceutical composition into an injection target without using an injection needle.

That is, for example,
in a case where the physiological activity exhibited by a nucleic acid when a liquid pharmaceutical composition containing the nucleic acid and a divalent cation at a total concentration of 0.0406 mM or more and less than 81.1 mM is injected into an injection target using a needle-equipped injector (this is referred to as embodiment C) is defined as "activity C", and
the physiological activity exhibited by a nucleic acid under the same conditions as in the embodiment C except that a liquid pharmaceutical composition containing the nucleic acid and a divalent cation at a total concentration of 0.0406 mM or more and less than 81.1 mM is injected into an injection target using an injector configured to inject a liquid pharmaceutical composition into an injection target without using an injection needle (this is referred to as embodiment A) is defined as "activity A",
activity C < activity A
may be satisfied.

In a case where the physiological activity exhibited by the nucleic acid is evaluated by a quantitative index (for example, an amount of expression, an amount of cellular immunity induced, an antitumor effect, a degree of maturity of immune cells, or the like), the quantitative index only needs to be appropriately set depending on the physiological activity exhibited by the nucleic acid.

In a case where the nucleic acid is DNA and contains a gene, examples of the quantitative index include an amount of mRNA produced using the DNA as a template and an amount of protein produced using the mRNA as a template (usually evaluated as an expression level of the gene).

In addition, for example, when the nucleic acid is mRNA, examples of the quantitative index include the amount of protein produced using the mRNA as a template.

That is, for example, in a case where the nucleic acid is DNA and contains a gene, and the quantitative index is an expression level of the gene,
an expression level of the gene when a liquid pharmaceutical composition containing a divalent cation at a total concentration of less than 0.0406 mM or 81.1 mM or more is injected into an injection target with an injector configured to inject a liquid pharmaceutical composition into an injection target without using an injection needle (this is referred to as embodiment B1) is defined as "expression level B1", and
an expression level of the gene when a liquid pharmaceutical composition is injected into an injection target in the same manner as described above under the same conditions as in the embodiment B1 except that the total concentration of the divalent cation is 0.0406 mM or more and less than 81.1 mM (this is referred to as embodiment A1) is defined as "expression level A1",
expression level B1 < expression level A1
is satisfied.

In a case where an expression level of the gene when a liquid pharmaceutical composition containing a divalent cation at a total concentration of 0.0406 mM or more and less than 81.1 mM is injected into an injection target using a needle-equipped injector (this is referred to as embodiment C1) is defined as "expression level C1", and
an expression level of the gene under the same conditions as in the embodiment C1 except that a liquid pharmaceutical composition containing a divalent cation at a total concentration of 0.0406 mM or more and less than 81.1 mM is injected into an injection target with an injector configured to inject a liquid pharmaceutical composition into an injection target without using an injection needle (this is referred to as embodiment A1) is defined as "expression level A1",
expression level C1 < expression level A1
may be satisfied.

In a case where the nucleic acid is, for example, a nucleic acid encoding a protein that induces cellular immunity, and the quantitative index is an induction amount of cellular immunity,
in a case where an induction amount of the cellular immunity when a liquid pharmaceutical composition containing a divalent cation at a total concentration of less than 0.0406 mM or 81.1 mM or more is injected into an injection target using an injector configured to inject a liquid pharmaceutical composition into an injection target without using an injection needle (this is referred to as embodiment B2) is defined to as "induction amount B1", and
an induction amount of the cellular immunity when a liquid pharmaceutical composition is injected into an injection target in the same manner as described above under the same conditions as in the embodiment B1 except that the total concentration of divalent cation is 0.0406 mM or more and less than 81.1 mM (this is referred to as embodiment A1) is defined as "induction amount A1",
induction amount B1 < induction amount A1
is satisfied.

This also applies to the induction amount of the cellular immunity in the injection target after restimulation (resensitization) of the protein that induces the cellular immunity.

The induction amount of the cellular immunity may be any amount as long as it indicates a degree of induction of cellular immunity, and may be, for example, the number of spots or the like when an ELISpot kit is used as in Examples described below.

In a case where an induction amount of the cellular immunity when a liquid pharmaceutical composition containing a divalent cation at a total concentration of 0.0406 mM or more and less than 81.1 mM is injected into an injection target using a needle-equipped injector (this is referred to as embodiment C1) is defined as "induction amount C1", and
an induction amount of the cellular immunity under the same conditions as in the embodiment C1 except that a liquid pharmaceutical composition containing a divalent cation at a total concentration of 0.0406 mM or more and less than 81.1 mM is injected into an injection target with an injector configured to inject a liquid pharmaceutical composition into an injection target without using an injection needle (this is referred to as embodiment A1) is defined as "induction amount A1",
induction amount C1 < induction amount A1
may be satisfied.

This also applies to the induction amount of the cellular immunity in the injection target after restimulation (resensitization) of the protein that induces the cellular immunity.

The method for evaluating the quantitative index can be appropriately selected, for example, depending on the physiological activity exhibited by the nucleic acid using the entirety or a part of an injection target after the liquid pharmaceutical composition is injected into the injection target.

For example, in a case where the injection target is an individual (living body), the nucleic acid is DNA containing a gene, and the expression level of the gene in the vicinity of an injection site (i.e., a part of the injection target) is evaluated as in Examples described below, after the liquid pharmaceutical composition is injected into the injection target, the vicinity of the injection site is obtained, and the method can be appropriately selected depending on the physiological activity exhibited by the nucleic acid.

Alternatively, as in Examples described below, the physiological activity exhibited by the nucleic acid can be evaluated by preparing a sample and measuring the amount of protein produced by a known method. In Examples described below, the protein produced is GFP protein, and thus the physiological activity is evaluated using fluorescent brightness as an index.

For example, as in Examples described below, in a case where the injection target is an individual (living body), the nucleic acid is a nucleic acid encoding a protein that induces cellular immunity, and the induction amount of cellular immunity is evaluated, after the liquid pharmaceutical composition is injected into the injection target, the induction amount of cellular immunity can be evaluated using a known method for evaluating the induction amount of cellular immunity using the individual (living body) itself (i.e., the entire injection target).

In the present embodiment, physiological activity obtained when the liquid pharmaceutical composition containing a nucleic acid exhibiting the physiological activity in an injection target is injected into the injection target is exhibited over a long period of time.

The timing at which the physiological activity is exhibited varies depending on the type of the nucleic acid, the injection target, the physiological activity exhibited by the nucleic acid in the injection target into which the nucleic acid is injected, and the like, and the physiological activity is usually exhibited after the elapse of a predetermined period from the time at which the liquid pharmaceutical composition is injected into the injection target.

The time (start time) at which the exhibition of the physiological activity is started is preferably as early as possible from the time at which the liquid pharmaceutical composition is injected into the injection target, and is, for example, 6 hours after the time at which the liquid pharmaceutical composition is injected into the injection target. Meanwhile, the time (end time) at which the exhibition of the physiological activity ends is preferably as late as possible from the time at which the liquid pharmaceutical composition is injected into the injection target, and is, for example, 1 day, 2 days, 3 days, 5 days, 7 days, or 14 days after the time at which the liquid pharmaceutical composition is injected into the injection target.

### (Divalent Cation)

When the total amount of the divalent cation in the liquid pharmaceutical composition according to the present embodiment is within a predetermined range, higher physiological activity is achieved when the liquid pharmaceutical composition is injected into an injection target using an injector configured to inject a liquid pharmaceutical composition into an injection target without using an injection needle, as compared with a case where a liquid pharmaceutical composition in the related art is injected.

The lower limit of the predetermined range is, for example, 0.0406 mM or more, 0.203 mM or more, 0.406 mM or more, 0.492 mM or more, 2.03 mM or more, 3.47 mM or more, 3.9 mM or more, 4.06 mM or more, 4.15 mM or more, 4.92 mM or more, 6.8 mM or more, 40.6 mM or more, or 49.2 mM or more.

The mechanism by which high physiological activity is achieved when the liquid pharmaceutical composition is injected into an injection target is not clear, but is presumed to be as follows. Note that the present disclosure is not limited to the following presumption.

In studies on nucleic acids and studies on chromosome condensation during cell division using an atomic force microscope (AFM), it is well known that nucleic acids have an action in which when a divalent cation such as a magnesium ion coexists with nucleic acids, charged crosslinking is induced and the nucleic acids aggregate.

Meanwhile, delivery of a nucleic acid into a cell by an injector configured to inject a liquid pharmaceutical composition into an injection target without using an injection needle is achieved by physical passage of the nucleic acid through a cell membrane by instantaneous injection and application of high energy. The structure of the cell membrane is a double-layer membrane of phospholipids, and it has been revealed that in a case where a nucleic acid physically passes through the cell membrane, steric hindrance such as a particle size greatly affects the passing efficiency, unlike a case where the nucleic acid passes through the cell membrane through adsorption to a receptor or the cell membrane. In fact, for direct passage of nanoparticles through a cell membrane, it has been reported that a small particle size is desirable.

From the above, it is presumed that the coexistence of a nucleic acid and a divalent cation induces particle formation of the nucleic acid and reduction in hydrodynamic diameter, whereby the delivery of the nucleic acid into a cell is made efficient. Then, it is presumed that the delivery of the nucleic acid into a cell in a case where an injector configured to inject a liquid pharmaceutical composition into an injection target without using an injection needle is used is made efficient, and high physiological activity is achieved.

Based on the above, it is presumed that in a case where the injection target is a cell or contains a cell, when the total concentration of the divalent cation is larger, the nucleic acid is likely to be formed into particles and the hydrodynamic diameter is likely to be reduced. As a result, the injection amount of the nucleic acid into the cell is likely to be increased and high physiological activity is likely to be achieved. Conversely, it is presumed that when the total concentration of the divalent cation is smaller, the nucleic acid is less likely to be formed into particles and the hydrodynamic diameter is less likely to be reduced. As a result, the injection amount of the nucleic acid into the cell is less likely to be increased and high physiological activity is less likely to be achieved.

The upper limit of the predetermined range is, for example, less than 81.1 mM, 49.2 mM or less, 40.6 mM or less, 6.8 mM or less, 4.92 mM or less, 4.15 mM or less, 4.06 mM or less, 3.9 mM or less, 3.47 mM or less, 2.03 mM or less, 0.492 mM or less, 0.406 mM or less, or 0.203 mM or less.

When the total concentration of the divalent cation is smaller, a load on the injection target due to the injection of the liquid pharmaceutical composition can be suppressed as much as possible. For example, in a case where the injection target is a cell or contains a cell, damage or death of the cell can be suppressed as much as possible.

The upper limit and the lower limit of the predetermined range may be a consistent combination selected from the examples of the upper limit and the examples of the lower limit described above in view of the reason for adopting the upper limit and the reason for adopting the lower limit. Specifically, the predetermined range may be as follows.

For example, the predetermined range may be 0.0406 mM or more and less than 81.1 mM, 0.0406 mM or more and 49.2 mM or less, 0.0406 mM or more and 40.6 mM or less, 0.0406 mM or more and 6.8 mM or less, 0.0406 mM or more and 4.92 mM or less, 0.0406 mM or more and 4.15 mM or less, 0.0406 mM or more and 4.06 mM or less, 0.0406 mM or more and 3.9 mM or less, 0.0406 mM or more and 3.47 mM or less, 0.0406 mM or more and 2.03 mM or less, 0.0406 mM or more and 0.492 mM or less, 0.0406 mM or more and 0.406 mM or less, or 0.0406 mM or more and 0.203 mM or less.

For example, the predetermined range may be 0.203 mM or more and less than 81.1 mM, 0.203 mM or more and 49.2 mM or less, 0.203 mM or more and 40.6 mM or less, 0.203 mM or more and 6.8 mM or less, 0.203 mM or more and 4.92 mM or less, 0.203 mM or more and 4.15 mM or less, 0.203 mM or more and 4.06 mM or less, 0.203 mM or more and 3.9 mM or less, 0.203 mM or more and 3.47 mM or less, 0.203 mM or more and 2.03 mM or less, 0.203 mM or more and 0.492 mM or less, or 0.203 mM or more and 0.406 mM or less.

For example, the predetermined range may be 0.406 mM or more and less than 81.1 mM, 0.406 mM or more and 49.2 mM or less, 0.406 mM or more and 40.6 mM or less, 0.406 mM or more and 6.8 mM or less, 0.406 mM or more and 4.92 mM or less, 0.406 mM or more and 4.15 mM or less, 0.406 mM or more and 4.06 mM or less, 0.406 mM or more and 3.9 mM or less, 0.406 mM or more and 3.47 mM or less, 0.406 mM or more and 2.03 mM or less, or 0.406 mM or more and 0.492 mM or less.

For example, the predetermined range may be 0.492 mM or more and less than 81.1 mM, 0.492 mM or more and 49.2 mM or less, 0.492 mM or more and 40.6 mM or less, 0.492 mM or more and 6.8 mM or less, 0.492 mM or more and 4.92 mM or less, 0.492 mM or more and 4.15 mM or less, 0.492 mM or more and 4.06 mM or less, 0.492 mM or more and 3.9 mM or less, 0.492 mM or more and 3.47 mM or less, or 0.492 mM or more and 2.03 mM or less.

For example, the predetermined range may be 2.03 mM or more and less than 81.1 mM, 2.03 mM or more and 49.2 mM or less, 2.03 mM or more and 40.6 mM or less, 2.03 mM or more and 6.8 mM or less, 2.03 mM or more and 4.92 mM or less, 2.03 mM or more and 4.15 mM or less, 2.03 mM or more and 4.06 mM or less, 2.03 mM or more and 3.9 mM or less, or 2.03 mM or more and 3.47 mM or less.

For example, the predetermined range may be 3.47 mM or more and less than 81.1 mM, 3.47 mM or more and 49.2 mM or less, 3.47 mM or more and 40.6 mM or less, 3.47 mM or more and 6.8 mM or less, 3.47 mM or more and 4.92 mM or less, 3.47 mM or more and 4.15 mM or less, 3.47 mM or more and 4.06 mM or less, or 3.47 mM or more and 3.9 mM or less.

For example, the predetermined range may be 3.9 mM or more and less than 81.1 mM, 3.9 mM or more and 49.2 mM or less, 3.9 mM or more and 40.6 mM or less, 3.9 mM or more and 6.8 mM or less, 3.9 mM or more and 4.92 mM or less, 3.9 mM or more and 4.15 mM or less, or 3.9 mM or more and 4.06 mM or less.

For example, the predetermined range may be 4.06 mM or more and less than 81.1 mM, 4.06 mM or more and 49.2 mM or less, 4.06 mM or more and 40.6 mM or less, 4.06 mM or more and 6.8 mM or less, 4.06 mM or more and 4.92 mM or less, or 4.06 mM or more and 4.15 mM or less.

For example, the predetermined range may be 4.15 mM or more and less than 81.1 mM, 4.15 mM or more and 49.2 mM or less, 4.15 mM or more and 40.6 mM or less, 4.15 mM or more and 6.8 mM or less, or 4.15 mM or more and 4.92 mM or less.

For example, the predetermined range may be 4.92 mM or more and less than 81.1 mM, 4.92 mM or more and 49.2 mM or less, 4.92 mM or more and 40.6 mM or less, or 4.92 mM or more and 6.8 mM or less.

For example, the predetermined range may be 6.8 mM or more and less than 81.1 mM, 6.8 mM or more and 49.2 mM or less, or 6.8 mM or more and 40.6 mM or less.

For example, the predetermined range may be 40.6 mM or more and less than 81.1 mM, or 40.6 mM or more and 49.2 mM or less.

For example, the predetermined range may be 49.2 mM or more and less than 81.1 mM.

Examples of the divalent cation include Mg2+, Ca2+, Ba2+, Cu2+, Fe2+, Mn2+, and Zn²⁺, and Mg²⁺, Ca²⁺, Mn²⁺, and Zn²⁺ are preferred. The divalent cation contained in the liquid pharmaceutical composition may be one type or a plurality of types.

The source of the divalent cation may be a "salt" which generates the divalent cation when dissolved in a solvent. Examples of the salt include chloride salts, sulfate salts, nitrate salts, aspartate salts, acetate salts, lactate salts, and gluconate salts. The source of the divalent cation may be in the form of a hydrate.

Examples of the Mg²⁺ source include magnesium chloride, magnesium sulfate, magnesium nitrate, magnesium aspartate, magnesium acetate, magnesium lactate, and magnesium gluconate. From the viewpoint of versatility, magnesium chloride, magnesium sulfate, magnesium nitrate, and magnesium aspartate are preferred.

Examples of the Ca²⁺ source include calcium chloride, calcium sulfate, calcium nitrate, calcium aspartate, calcium acetate, calcium lactate, and calcium gluconate. From the viewpoint of versatility, calcium chloride is preferred.

Examples of the Ba²⁺ source include barium chloride, barium sulfate, barium nitrate, barium aspartate, barium acetate, barium lactate, and barium gluconate.

Examples of the Cu²⁺ source include copper(II) chloride, copper(II) sulfate, copper(II) nitrate, copper(II) aspartate, copper(II) acetate, copper(II) lactate, and copper(II) gluconate.

Examples of the Fe²⁺ source include iron(II) chloride, iron(II) sulfate, iron(II) nitrate, iron(II) aspartate, iron(II) acetate, iron(II) lactate, and iron(II) gluconate.

Examples of the Mn²⁺ source include manganese(II) chloride, manganese(II) sulfate, manganese(II) nitrate, manganese(II) aspartate, manganese(II) acetate, manganese(II) lactate, and manganese(II) gluconate. From the viewpoint of versatility, manganese(II) sulfate is preferred.

Examples of the Zn²⁺ source include zinc chloride, zinc sulfate, zinc nitrate, zinc aspartate, zinc acetate, zinc lactate, and zinc gluconate.

### (Injection Target)

The injection target in the present embodiment may be, for example, one or more selected from the group consisting of a cell, a cell sheet, a cell cluster, a tissue, an organ (skin, a body part, or the like), an organ system, an individual (living body), and the like, and is not limited. Any of an in vitro system, an in vivo system, and an ex vivo system may be adoptable. The cell cluster may be a cell cluster obtained by three-dimensional culture, and the organ (skin, body part, or the like) may be an organoid.

When injection is performed to the injection target, the injection may be performed to a lower layer included in the injection target. That is, for example, when an individual (living body) is an injection target, injection may be performed to a tissue included in the individual (living body), may be performed to a cell included in the individual (living body), or may be performed to both of the tissue and the cell. When a tissue is an injection target, injection may be performed to a cell included in the tissue, may be performed to an intercellular matrix included in the tissue, or may be performed to both of the cell and the intercellular matrix.

When the injection target in the present embodiment is one or more selected from the group consisting of a cell, a cell sheet, a cell cluster, a tissue, an organ (skin, a body part, or the like), and an organ system, the injection target may be one or more selected from the group consisting of a cell, a cell sheet, a cell cluster, a tissue, an organ (skin, a body part, or the like), and an organ system existing in an individual (living body), or one or more selected from the group consisting of a cell, a cell sheet, a cell cluster, a tissue, an organ (skin, a body part, or the like), and an organ system not existing in an individual (living body) (for example, in a state extracted or separated from the individual (living body) or a state produced outside the individual (living body)).

The injection target in the present embodiment may be one or more selected from the group consisting of a cell, a cell sheet, a cell cluster, a tissue, an organ (skin, a body part, or the like), and an organ system derived from a stem cell such as an iPS cell (induced pluripotent stem cell), and may be present in an individual (living body) or not present in an individual (living body) (for example, in a state extracted or separated from the individual (living body), or a state produced outside the individual (living body)). The cell cluster may be a cell cluster obtained by three-dimensional culture, and the organ (skin, body part, or the like) may be an organoid.

The individual (living body) is preferably a mammalian individual (living body). The mammal is not particularly limited, but examples of the mammal include humans and mammals other than the human. Examples of the mammal other than the human include mouse, rat, guinea pig, hamster, cow, goat, sheep, pig, monkey, dog, and cat.

Regardless of what the injection target of the present embodiment is among the examples described above, when injection is performed to a cell, the injection may be performed to the cytoplasm of the cell, the injection may be performed to the cell nucleus of the cell, or the injection may be performed to both the cytoplasm and the cell nucleus of the cell.

The injection target of the present embodiment is not particularly limited, but is preferably one or more selected from the group consisting of an intradermal part, a subcutaneous part, and a muscle layer located underneath the skin of a mammalian individual (living body). In this case, a method may be employed in which the liquid pharmaceutical composition is ejected from the injector toward the surface of the skin and is injected into the skin, and the liquid pharmaceutical composition is injected into one or more selected from the group consisting of an intradermal part in the skin, a subcutaneous part, and a muscle layer located underneath the skin.

### (Liquid Pharmaceutical Composition)

In the liquid pharmaceutical composition according to the present embodiment, the nucleic acid and the divalent cation may be in a form in which they are dissolved in a pharmacologically acceptable liquid (solvent). Examples of the pharmacologically acceptable liquid include water (for example, water for injection), and a buffer solution containing a phosphate (for example, a phosphate buffer solution, or phosphate buffered saline (PBS) (which may be PBS(+) or PBS(-)).

In the present disclosure, "dissolution" may be "suspension" or "emulsification", "solution" may be "suspension" or "emulsion", and "solvent" may be "suspending agent" or "emulsifier".

The liquid pharmaceutical composition according to the present embodiment contains the nucleic acid and the divalent cation, and can be mixed with a non-toxic carrier for a pharmaceutical composition. Examples of the carrier include glucose, lactose, sucrose, starch, mannitol, dextrin, fatty acid glyceride, poly(ethylene glycol), hydroxyethyl starch, ethylene glycol, polyoxyethylene sorbitan fatty acid esters, amino acids, gelatin, albumin, water, and physiological saline. As necessary, a commonly used additive, such as a stabilizer, a wetting agent, an emulsifier, a binder, or an isotonizing agent, can be appropriately added.

The content of the nucleic acid relative to the total amount of the liquid pharmaceutical composition according to the present embodiment can be appropriately set based on the type of the nucleic acid, the injection target, the physiological activity exhibited by the nucleic acid in the injection target into which the nucleic acid has been injected, and the like. The content of the nucleic acid can be appropriately set in consideration of the content of the divalent cation.

The injection amount of the liquid pharmaceutical composition into the injection target can be appropriately set based on the content and type of the nucleic acid, the injection target, the physiological activity exhibited by the nucleic acid in the injection target into which the nucleic acid has been injected, and the like. The injection amount can be appropriately set in consideration of the injection amount of the divalent cation.

The pH of the liquid pharmaceutical composition according to the present embodiment is not particularly limited as long as, when the liquid pharmaceutical composition is injected into an injection target by an injector configured to inject the liquid pharmaceutical composition into the injection target without using an injection needle, the nucleic acid exhibits physiological activity in the injection target and is stably present therein, and there is no adverse effect such as destruction of the injection target.

The liquid pharmaceutical composition according to the present embodiment may be in the form of a vaccine.

The liquid pharmaceutical composition according to the present embodiment is preferably used for the purpose of inducing cellular immunity. In this case, the injection target may be an injection target in which cellular immunity can be induced (for example, an individual (living body)). The nucleic acid may be a nucleic acid encoding a protein that induces cellular immunity. In Examples described below, ovalbumin (OVA) is used as the protein inducing cellular immunity, but the cellular immunity is an immune response to a foreign substance, and the protein inducing the cellular immunity is not limited only to OVA.

The induction of cellular immunity may be a secondary phenomenon resulting from induction of cellular immunity. For example, the induction of cellular immunity may be induction of production of interferon gamma (IFN-γ).

### (Injector)

The injector used in the present embodiment may be an injector capable of injecting the liquid pharmaceutical composition into the injection target without using an injection needle. That is, the liquid pharmaceutical composition may be injected into an injection target by an injector configured to inject a liquid pharmaceutical composition into an injection target without using an injection needle.

In the injector according to the present embodiment, the term "tip end side" means a side on which an ejection port through which the liquid pharmaceutical composition is ejected from the injector is disposed, and the term "base end side" means a side opposite to the tip end side in the injector, and these terms do not limitedly indicate a specific portion or position.

In the injector according to the present embodiment, a drive section applies ejection energy to eject the liquid pharmaceutical composition toward the injection target. The "ejection" by the injector according to the present embodiment is realized by using the ejection energy by the drive section, pressurizing the liquid pharmaceutical composition that is stored in the storage section by the pressurization section, and thus causing the liquid pharmaceutical composition to flow through a flow path of the storage section. The ejection energy may be ejection energy to be used in a typical injector, and for example, combustion energy of an explosive or the like, generation energy of a gas generating agent or the like, electric energy of a piezoelectric element or the like, or mechanical energy of a spring or the like may be used, or energy obtained by appropriately combining these forms of energy may be used.

In a case where the combustion energy of the explosive is used as the ejection energy, it is preferable to use, for example, any one of an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), and an explosive containing aluminum and iron oxide (AFO), or an explosive composed of a plurality of these explosives in combination. Such an explosive is characterized in that the combustion product thereof is gas at a high temperature but does not contain a gas component at normal temperature, and hence, the combustion product is condensed immediately after the ignition. Thus, during a pressurization process for ejection of the liquid pharmaceutical composition, the temperature of the combustion product at the time of pressurization can be shifted by the combustion of an ignition agent to around the normal temperature in a short period of time after the pressure applied to the liquid pharmaceutical composition reaches a first peak ejection force.

When the generation energy by the gas generating agent is used as the ejection energy, the gas generating agent may be a single base smokeless explosive (for example, a single base smokeless explosive containing 98 mass% of nitrocellulose, 0.8 mass% of diphenylamine, and 1.2 mass% of potassium sulfate), or any of various gas generating agents used in gas generators for air bags and gas generators for seat belt pretensioners.

In the injector according to the present embodiment, the pressurization section pressurizes the liquid pharmaceutical composition that is stored in the storage section during the operation, which causes the liquid pharmaceutical composition to be ejected from the ejection port toward the injection target.

The pressurization by the pressurization section is not particularly limited as long as a system is not destroyed, for example, like the destruction of the storage section, and pressurization conditions of an ordinary injector may be employed.

In this context, the pressure means a pressure in the storage section. The method for measuring the pressure is not particularly limited, and for example, measurement can be performed as follows. That is, as in the measurement method described in JP 2005-21640 A, measurement is performed by a method in which an ejection force is applied in a dispersed manner to a diaphragm of a load cell disposed downstream of a nozzle and an output from the load cell is collected by a data collection device via a detection amplifier and is stored as an ejection force (N) per unit time. When the ejection pressure measured in this manner is divided by the area of an ejection port 31a of the injector, the ejection pressure is calculated. The measurement value obtained by the internal pressure measurement of the storage section is equivalent to the ejection pressure, and the ejection pressure can be regarded as the pressure inside the storage section.

The ejection energy by the drive section is transmitted to a plunger through a piston, and the plunger slides in the storage section, whereby the liquid pharmaceutical composition that is stored in the storage section is pushed out along the flow path formed in the nozzle section, and finally ejected from the ejection port toward the injection target.

The liquid pharmaceutical composition may or may not be stored in the storage section from the beginning. In a case where the liquid pharmaceutical composition is not stored in the storage section, the liquid pharmaceutical composition can be stored in the storage section by being aspirated into the storage section through a nozzle including an ejection port. Employing the configuration that requires the storing operation into the storage section in this manner allows any necessary liquid pharmaceutical composition to be injected into the injection target. For this reason, in the injector according to the present embodiment, a syringe section and an injector body may be detachably configured.

In the present embodiment, the injection of the liquid pharmaceutical composition into the injection target by the injector configured to inject a liquid pharmaceutical composition into an injection target without using an injection needle may be injection into the injection target by jet injection.

The term "jet injection" in the present disclosure refers to injection without using an injection needle, characterized in that a liquid pharmaceutical composition containing a nucleic acid and a divalent cation and having a total concentration of the divalent cation of 0.0406 mM or more and less than 81.1 mM is ejected from an ejection port toward an injection target to form a through hole penetrating the boundary between the inside and the outside of the injection target, and a high-pressure ultrafine liquid flow capable of injecting the liquid pharmaceutical composition into the injection target is generated through the through hole. For example, in a case where the injection target is a mammalian individual (living body), the jet injection refers to injection characterized by generating a high-pressure ultrafine liquid flow capable of penetrating the skin of the mammalian individual (living body), for example.

The injector according to the present embodiment can be obtained by storing the liquid pharmaceutical composition in the storage section in a typical injector that can be used to inject an injection solution (such as the liquid pharmaceutical composition) into an injection target without using an injection needle. Examples of such a typical injector include the injector described in WO 2019/156238, the injector described in WO 2019/156239, the injector described in WO 2019/156237, and the injector described in JP 5989039 B. These are injectors capable of jet injection, but there are many other commercially available injectors (needleless injectors) capable of the jet injection. Examples thereof include Straits (Pharmajet), Tropis (Pharmajet), Vitajet (Bioject Medical Technologies Inc.), Biojector 2000 (Bioject Medical Technologies Inc.), Bioject Zetajet (Bioject Medical Technologies Inc.), Glide (Glide Pharma), MediJector Vision (Antares Inc.), Sumaval DosePro (Zogenix Inc.), SQ Pen (Bespak), Injex (Equidyne), and hyaluronic acid syringe (BEAUTTO, Amazon Standard Identification Number (ASIN): B08NCHTRHZ).

With reference to the drawings, an injector 1 (needleless injector) will be described below as an example of the injector according to the present embodiment. Note that a configuration according to the following embodiment is provided as an example, and the disclosure is not limited to the configuration according to the present embodiment. The terms "tip end side" and "base end side" are used as terms indicating a relative positional relationship in a longitudinal direction of the injector 1. The term "tip end side" indicates a side close to the tip end of the injector 1 to be described below, that is, a position close to an ejection port 31a, and the term "base end side" indicates a direction opposite to the "tip end side" in the longitudinal direction of the injector 1, that is, a direction toward a side of a drive section 7. The present example is an example in which combustion energy of an explosive ignited by an ignition device is used as ejection energy for pressurization, but the present embodiment is not limited to this example.

### (Configuration of Injector 1)

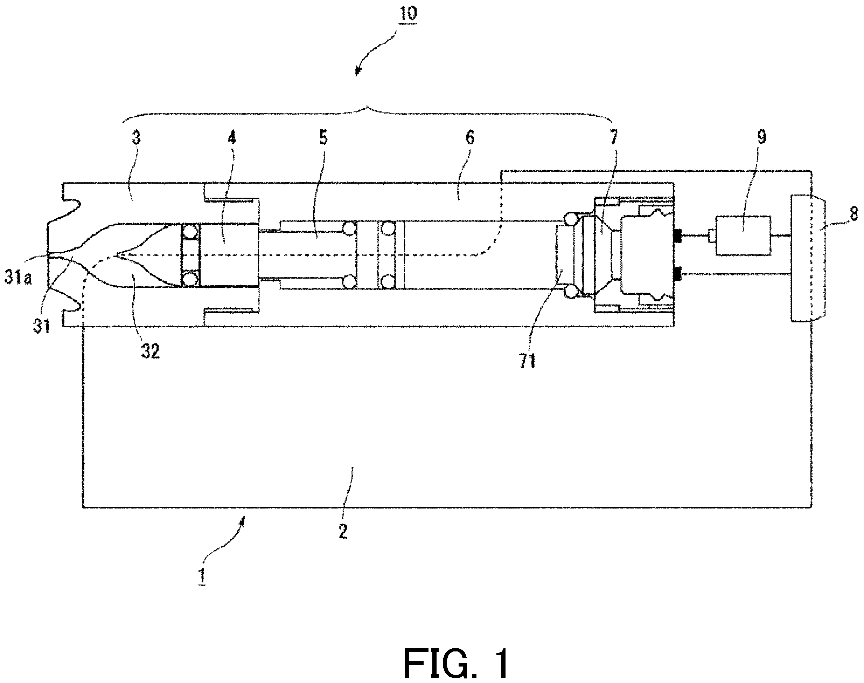

FIG. 1 is a cross-sectional view of the injector 1, taken along the longitudinal direction thereof, illustrating a schematic configuration of the injector 1. The injector 1 is formed by attaching an injector assembly 10 to a housing (injector housing) 2. The injector assembly 10 includes a subassembly including a syringe section 3 and a plunger 4 and a subassembly including an injector body 6, a piston 5, and a drive section 7, and the subassemblies are integrally assembled.

As described above, the injector assembly 10 is configured to be attachable and detachable to and from the housing 2. A storage section 32 formed between the syringe section 3 and the plunger 4 included in the injector assembly 10 is filled with the liquid pharmaceutical composition. The injector assembly 10 is a unit that is disposed after every ejection of the liquid pharmaceutical composition. Meanwhile, a battery 9 that supplies power to an igniter 71 included in the drive section 7 of the injector assembly 10 is included on the housing 2 side. The power supply from the battery 9 is performed between an electrode on the housing 2 side and an electrode on the drive section 7 side of the injector assembly 10 through wiring lines, when a user performs an operation of pressing a button 8 provided on the housing 2. The electrode on the housing 2 side and the electrode on the drive section 7 side of the injector assembly 10 have shapes and positions designed to come into contact with each other automatically when the injector assembly 10 is attached to the housing 2. The housing 2 is a unit that can be repeatedly used as long as there is power remaining in the battery 9 that can be supplied to the drive section 7. Note that when the battery 9 runs out of power in the housing 2, the housing 2 may be continued to be used with only the battery 9 exchanged.

The details of the injector assembly 10 will next be described. First of all, a description is given on the subassembly including the syringe section 3 and the plunger 4. In the syringe section 3, the storage section 32 is formed as a space in which the liquid pharmaceutical composition can be stored. More specifically, as illustrated in FIG. 1, the plunger 4 is disposed slidably along an inner wall surface extending in the axial direction of the syringe section 3, and the storage section 32 is defined by the inner wall surface of the syringe section 3 and the plunger 4. Additionally, the syringe section 3 includes a nozzle section 31 communicating with the storage section 32, and the nozzle section 31 is provided with the ejection port 31a on the tip end side. The nozzle section 31 is a flow path whose cross-sectional area gradually decreases from the storage section 32 side toward the ejection port 31a side, and the flow path is configured to guide the liquid pharmaceutical composition that is filled in the storage section 32 to the ejection port 31a. In the example illustrated in FIG. 1, the plunger 4 has a shape on the tip end side that substantially matches the shape of the nozzle section 31.

Next will be described the subassembly including the injector body 6, the piston 5, and the drive section 7. The piston 5 is made of metal, for example, and is configured to be pressurized by a combustion product (combustion gas) generated by the igniter 71 of the drive section 7 and to slide in a through hole formed in the injector body 6. The injector body 6 is a substantially cylindrical member, and the piston 5 is contained therein slidably along the inner wall surface extending in the axial direction thereof. The piston 5 may be formed of a resin, and in such a case, a metal may be used in combination for a portion required to have heat resistance and pressure resistance. Additionally, as illustrated in FIG. 1, the piston 5 is integrally connected with the plunger 4.

The drive section 7 will next be described. As illustrated in FIG. 1, the drive section 7 is fixed on the base end side with respect to the through hole in the injector body 6. The drive section 7 includes the igniter 71, which is an electric igniter. The igniter 71 is disposed to face the interior of the through hole in the injector body 6 and contains an ignition agent therein. As the ignition agent, various types of explosives can be employed as described above. The ignition agent can be contained in an explosive cup formed of an appropriate thin metal, for example.

How the injector 1 having the configuration described above is operated will next be described. As illustrated in FIG. 1, in a state where the injector assembly 10 is attached to the housing 2, the liquid pharmaceutical composition is aspirated from the ejection port 31a of the nozzle section 31. This allows the liquid pharmaceutical composition to be filled in the storage section 32. In this state, for example, with the ejection port 31a of the injector 1 being in contact with the injection target, when a user performs an operation of pressing the button 8 provided on the housing 2, this serves as a trigger and actuation power is supplied from the battery 9 to the igniter 71 of the drive section 7, and thus, the igniter 71 is activated. When the igniter 71 is activated, the ignition agent is ignited and thus combusted, and combustion products (flame, combustion gas, and the like) are generated. As a result, an explosive cup of the igniter 71 is ruptured, for example, and the combustion gas of the ignition agent is released into the through hole in the injector body 6. Thus, the pressure in the through hole of the injector body 6 rapidly increases, and the piston 5 is pressed toward the tip end side of the injector body 6. As a result, the piston 5 slides along the inner wall surface of the through hole in the injector body 6 toward the tip end side. As described above, because the plunger 4 is connected integrally with the piston 5, the plunger 4 also slides along the inner wall surface of the syringe section 3 in conjunction with the piston 5. That is, with the plunger 4 pushed toward the nozzle section 31 located on the tip end side of the syringe section 3, the volume of the storage section 32 storing the liquid pharmaceutical composition is reduced, and the liquid pharmaceutical composition is rapidly pressurized. As a result, the liquid pharmaceutical composition that is filled in the storage section 32 is pushed into the nozzle section 31 and is ejected from the ejection port 31a at a high pressure. Thus, the liquid pharmaceutical composition can be injected into the injection target.

Although an additional explosive component is not particularly provided in the injector body 6 illustrated in FIG. 1, a gas generating agent or the like, which is combusted by a combustion product generated by the explosive combustion at the igniter 71 and generates gas, may be provided in the igniter 71 or the through hole of the injector body 6 to adjust a change in pressure applied to the liquid pharmaceutical composition through the piston 5. The configuration in which the gas generating agent is provided in the igniter 71 is a known technique as disclosed in WO 01-031282, JP 2003-25950 A, and the like. One example of the gas generating agent is a single base smokeless explosive containing 98 mass% of nitrocellulose, 0.8 mass% of diphenylamine, and 1.2 mass% of potassium sulfate. Various types of gas generating agents used in a gas generator for an air bag and a gas generator for a seat belt pretensioner may be used. A combustion completion time period of the gas generating agent can be changed by adjusting the dimensions, size, shape, and particularly, surface shape of the gas generating agent when provided in the through hole, which allows a change in pressure applied to the liquid pharmaceutical composition to be adjusted to a desired change, that is, a change that causes the liquid pharmaceutical composition to appropriately reach the injection target. In the present embodiment, the gas generating agent to be used as necessary is also included in the drive section 7. In the present embodiment, the plunger 4 and the piston 5 constitute the "pressurization section".

Another embodiment of the present disclosure is
an injector configured to inject a liquid pharmaceutical composition into an injection target without using an injection needle, the injector including:
a storage section containing the liquid pharmaceutical composition,
a nozzle section communicating with the storage section, the nozzle section having an ejection port for ejecting the liquid pharmaceutical composition toward the injection target, and
a pressurization section for pressurizing the liquid pharmaceutical composition contained in the storage section during an operation, thereby ejecting the liquid pharmaceutical composition from the ejection port toward the injection target, wherein
the liquid pharmaceutical composition contains a nucleic acid and a divalent cation, and a total concentration of the divalent cation is 0.0406 mM or more and less than 81.1 mM.

The injector according to the present embodiment is the injector configured to inject a liquid pharmaceutical composition into an injection target without using an injection needle described in the above embodiment, in which the liquid pharmaceutical composition according to the above embodiment is stored in the storage section. The description of the above embodiment is incorporated in the description of the present embodiment.

Another embodiment of the present disclosure is
a method for injecting a liquid pharmaceutical composition into an injection target, the liquid pharmaceutical composition containing a nucleic acid and a divalent cation and having a total concentration of the divalent cation of 0.0406 mM or more and less than 81.1 mM, the method including injecting the liquid pharmaceutical composition into the injection target using an injector configured to inject the liquid pharmaceutical composition into the injection target without using an injection needle.

The method according to the present embodiment is a method for injecting the liquid pharmaceutical composition according to the above embodiment into an injection target using the injector according to the above embodiment configured to inject the liquid pharmaceutical composition into the injection target without using an injection needle. The description of the above embodiment is incorporated in the description of the present embodiment.

When the liquid pharmaceutical composition is injected into an injection target by the method according to the present embodiment, the physiological activity of the nucleic acid contained in the liquid pharmaceutical composition is exhibited in the injection target as described in the above embodiment.

Thus, the method according to the present embodiment may be a method for allowing a nucleic acid to exhibit physiological activity in an injection target, the method including
injecting a liquid pharmaceutical composition containing a nucleic acid and a divalent cation and having a total concentration of the divalent cation of 0.0406 mM or more and less than 81.1 mM into an injection target using an injector configured to inject the liquid pharmaceutical composition into the injection target without using an injection needle.

### EXAMPLES

Examples will be described below, but none of the examples should be construed as limiting the present disclosure.

The following experiments on animals were conducted in the Institute of Experimental Animal Sciences, Faculty of Medicine, Osaka University and were carried out in accordance with Osaka University Regulations on Animal Experiments defined by the Animal Experiments Committee, Osaka University.

### [Test Example 1]

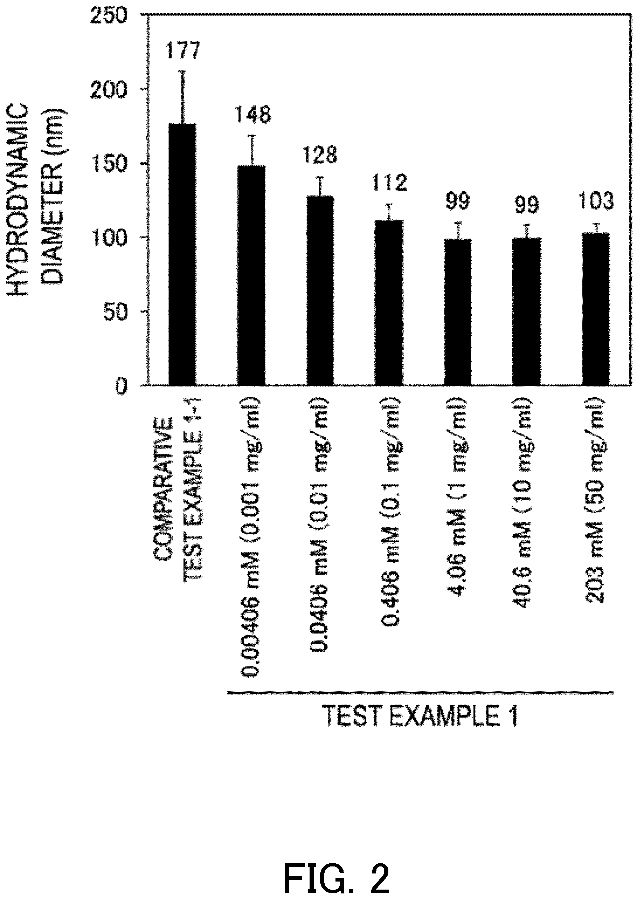

PBS-tablet (available from Takara Bio Inc.) (PBS(-)) was dissolved in water for injection (available from Otsuka Pharmaceutical Co., Ltd.) to prepare 2-fold PBS, which was sterilized with a filter. Magnesium sulfate (MgSO₄·7H₂O) (Nacalai Tesque) was dissolved in the prepared 2-fold PBS in such a manner that each of the final concentrations shown in FIG. 2 was achieved, which was sterilized with a filter. Thereafter, the resultant solution was mixed with CMV-DASHER-GFP (available from ATUM) (solvent: TE) (1.0 mg/mL), which was a plasmid DNA containing a GFP gene, at a ratio of 1:1 to prepare a liquid pharmaceutical composition. Specifically, the liquid pharmaceutical composition was prepared in such a manner that the final concentration of the plasmid DNA containing a GFP gene was 0.5 mg/mL and the final concentration of Mg²⁺ (the final concentration of MgSO₄·7H₂O is shown in parentheses) was 0.00406 mM (0.001 mg/ml), 0.0406 mM (0.01 mg/ml), 0.406 mM (0.1 mg/ml), 4.06 mM (1 mg/ml), 40.6 mM (10 mg/ml), or 203 mM (50 mg/ml).

Using the prepared liquid pharmaceutical composition, a hydrodynamic diameter was measured by dynamic light scattering (DLS) (Malvern Panalytical Ltd., NANO-ZS). An average hydrodynamic diameter was measured by preparing a measurement sample with a volume of 1.0 mL and using a plastic cuvette. Values calculated from scattering intensities were used as measurement results, and an average and a standard deviation were determined based on at least five different measurements.

### [Comparative Test Example 1-1]

PBS-tablet (available from Takara Bio Inc.) (PBS(-)) was dissolved in water for injection (available from Otsuka Pharmaceutical Co., Ltd.) to prepare 2-fold PBS, which was sterilized with a filter. Thereafter, the resultant solution was mixed with CMV-DASHER-GFP (available from ATUM) (solvent: TE) (1.0 mg/mL), which was a plasmid DNA containing a GFP gene, at a ratio of 1:1 to prepare a liquid pharmaceutical composition. Specifically, a liquid pharmaceutical composition was prepared in which the final concentration of the plasmid DNA containing a GFP gene was 0.5 mg/mL and the final concentration of Mg²⁺ was 0. The other conditions were the same as in Test Example 1.

The results are shown in FIG. 2. In Comparative Test Example 1-1, the hydrodynamic diameter was 177 nm, whereas in Test Example 1, a smaller hydrodynamic diameter was exhibited along with an increase in Mg²⁺ concentration. When the Mg²⁺ concentration was 1 mg/mL (the Mg²⁺ concentration was 4.06 mM) or more, the hydrodynamic diameter was about 100 nm. It is considered that this is because electrical crosslinking proceeded inside the nucleic acid with an increase in Mg²⁺ concentration and aggregation proceeded in the molecule. In a case where the Mg²⁺ concentration was 1 mg/mL (the Mg²⁺ concentration was 4.06 mM) or more, it is considered that the electrical crosslinking of the nucleic acid had sufficiently occurred, not leading to a further decrease in the hydrodynamic diameter.

### [Comparative Test Example 1-2]

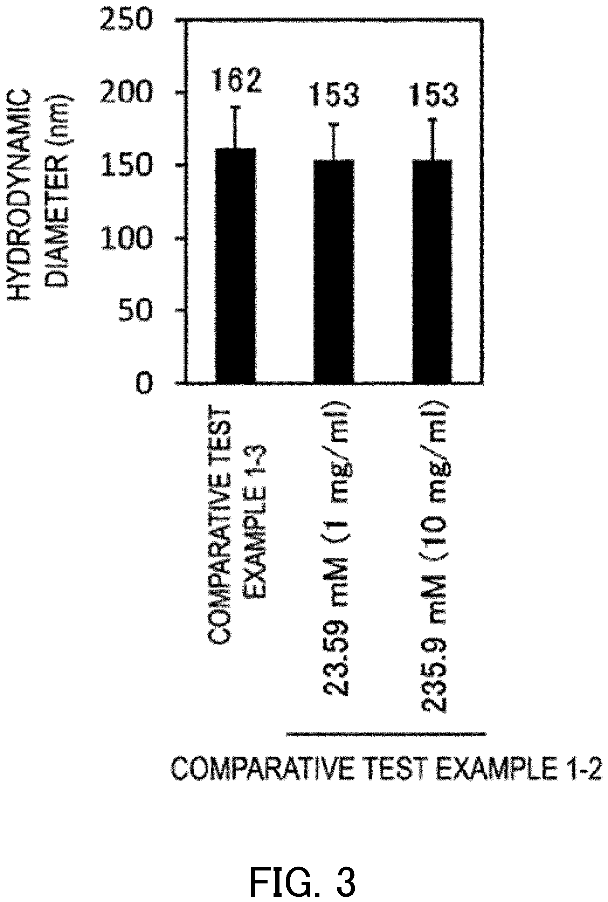

The same procedure as in Test Example 1 was repeated except that lithium chloride (LiCl) (Nacalai Tesque) was used instead of magnesium sulfate (MgSO₄·7H₂O) in such a manner that final concentrations shown in FIG. 3 were achieved. Specifically, liquid pharmaceutical compositions were prepared in such a manner that the final concentration of Li⁺ (the final concentration of LiCl is shown in parentheses) was 23.59 mM (1 mg/ml) or 235.9 mM (10 mg/ml).

### [Comparative Test Example 1-3]

PBS-tablet (available from Takara Bio Inc.) (PBS(-)) was dissolved in water for injection (available from Otsuka Pharmaceutical Co., Ltd.) to prepare 2-fold PBS, which was sterilized with a filter. Thereafter, the resultant solution was mixed with CMV-DASHER-GFP (available from ATUM) (solvent: TE) (1.0 mg/mL), which was a plasmid DNA containing a GFP gene, at a ratio of 1:1 to prepare a liquid pharmaceutical composition. Specifically, the final concentration of the plasmid DNA containing a GFP gene was adjusted to 0.5 mg/mL, and a liquid pharmaceutical composition having a final concentration of Li⁺ of 0 was prepared. The other conditions were the same as in Test Example 1. Comparative Test Example 1-3 is substantially the same as Comparative Test Example 1-1.

The results are shown in FIG. 3. In Comparative Test Example 1-3, the hydrodynamic diameter was 162 nm, whereas in Comparative Test Example 1-2, even when the Li⁺ concentration increased, the hydrodynamic diameter changed only up to 153 nm. It is considered that this is because, unlike the case of Mg²⁺, which is a divalent cation, Li⁺, which is a monovalent cation, did not cause electrical crosslinking of the nucleic acid, and thus the nucleic acid was not formed into particles.

The above results revealed that in a case where Mg²⁺ is present in the nucleic acid solution, electrical crosslinking occurs in the nucleic acid and the nucleic acid is formed into particles.

### [Test Example 2]

### (Preparation of Liquid Pharmaceutical Composition)

PBS-tablet (available from Takara Bio Inc.) (PBS(-)) was dissolved in water for injection (available from Otsuka Pharmaceutical Co., Ltd.) to prepare 2-fold PBS, which was sterilized with a filter. Magnesium chloride (MgCl₂·6H₂O, Nacalai Tesque) was dissolved in the prepared 2-fold PBS to achieve a concentration twice as high as that when injected into an 11-week-old male balb/c mouse (CLEA Japan, Inc.) as a test animal, followed by filter sterilization. Thereafter, the resultant solution was mixed with CMV-DASHER-GFP (available from ATUM) (solvent: TE) (1.0 mg/mL), which was a plasmid DNA containing a GFP gene, at a ratio of 1:1 to prepare a liquid pharmaceutical composition. Specifically, a liquid pharmaceutical composition was prepared in such a manner that the final concentration of the plasmid DNA containing a GFP gene was 0.5 mg/mL_{,} and the final concentration of Mg²⁺ (the final concentrations of MgCl₂·6H₂O are shown in parentheses) was 0.492 mM (0.1 mg/ml), 4.92 mM (1 mg/ml), or 49.2 mM (10 mg/ml).

### (Injection into Mouse)

A mouse was anesthetized by aspiration with isoflurane, and then its back was clipped using animal hair clippers. Thereafter, the back was disinfected with alcohol, and the liquid pharmaceutical composition was injected into a plurality of sites of the back using the following injector. 20 µL of the liquid pharmaceutical composition per site was injected. The injector used is the injector illustrated in FIG. 1 (needleless injector), with a nozzle diameter of 0.1 mm. 25 mg of ZPP was used as an ignition agent for the injector, and 40 mg of a single base smokeless explosive (containing 98 mass% of nitrocellulose, 0.8 mass% of diphenylamine, and 1.2 mass% of potassium sulfate) was used as a gas generating agent.

### (Evaluation of Gene Expression)

One day after the injection, the mouse was euthanized. Thereafter, a piece of skin having a thickness sufficient for microscopic observation was collected from the back, attached to a Matsunami glass bottom dish, and then observed with a fluorescent microscope BZ-X710 (available from Keyence Corporation). A bright field image (exposure time: 1/350 s) and a fluorescent image of the GFP channel (exposure time: 1/20 s) were acquired, and as necessary, a superimposed image (bright field image + fluorescent image) was acquired.

### [Comparative Test Example 2]

PBS-tablet (available from Takara Bio Inc.) (PBS(-)) was dissolved in water for injection (available from Otsuka Pharmaceutical Co., Ltd.) to prepare 2-fold PBS, which was sterilized with a filter. Thereafter, the resultant solution was mixed with CMV-DASHER-GFP (available from ATUM) (solvent: TE) (1.0 mg/mL), which was a plasmid DNA containing a GFP gene, at a ratio of 1:1 to prepare a liquid pharmaceutical composition. Specifically, the final concentration of the plasmid DNA containing a GFP gene was adjusted to 0.5 mg/mL to prepare a liquid pharmaceutical composition having a Mg²⁺ concentration of 0. The other conditions were the same as in Test Example 2.

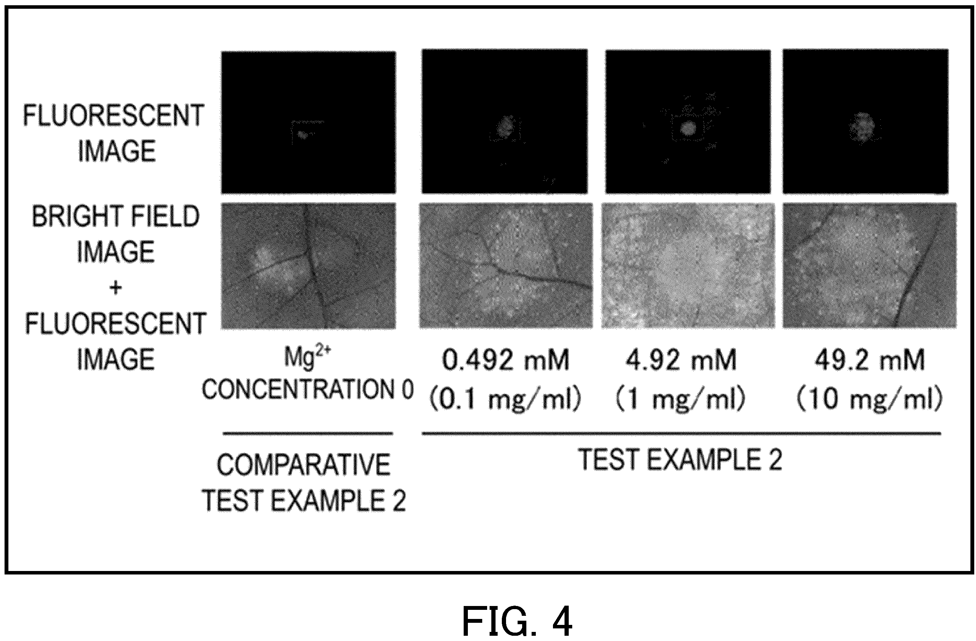

The results are shown in FIG. 4. As compared with Comparative Test Example 2, clearly strong fluorescence derived from GFP was observed at any Mg²⁺ concentration in Test Example 2.

From the results, it was confirmed that in a case where Mg²⁺ is present in the nucleic acid solution, the efficiency of gene transfer by the injector (needleless injector) illustrated in FIG. 1 is improved to bring about high gene expression.

### [Test Example 3]

PBS-tablet (available from Takara Bio Inc.) (PBS(-)) was dissolved in water for injection (available from Otsuka Pharmaceutical Co., Ltd.) to prepare 2-fold PBS, which was sterilized with a filter. Magnesium sulfate (MgSO₄·7H₂O, Nacalai Tesque) was dissolved in the prepared 2-fold PBS to achieve a concentration twice as high as that when injected into 11- to 13-week-old male balb/c mice (CLEA Japan, Inc.) as test animals, followed by filter sterilization. Thereafter, the resultant solution was mixed with CMV-DASHER-GFP (available from ATUM) (solvent: TE) (1.0 mg/mL), which was a plasmid DNA containing a GFP gene, at a ratio of 1:1 to prepare a liquid pharmaceutical composition. Specifically, a liquid pharmaceutical composition was prepared in such a manner that the final concentration of the plasmid DNA containing a GFP gene was 0.5 mg/mL and the final concentration of Mg²⁺ (the final concentration of MgSO₄·7H₂O is shown in parentheses) was 0.00406 mM (0.001 mg/ml), 0.0406 mM (0.01 mg/ml), 0.203 mM (0.05 mg/ml), 0.406 mM (0.1 mg/ml), 4.06 mM (1 mg/ml), 40.6 mM (10 mg/ml), 81.1 mM (20 mg/ml), or 203 mM (50 mg/ml).

Injection into mice was performed in the same manner as in Test Example 2. A bright field image (exposure time: 1/350 s) and a fluorescent image of the GFP channel (exposure time: 1/20 s) were acquired, and a superimposed image was acquired as necessary. Then, for analysis of gene expression, the fluorescent image was analyzed using an analysis application (Hybrid Cell Count) (available from Keyence Corporation). The analysis was performed under the conditions of a brightness setting of 10 and a region designation of off.

### [Comparative Test Example 3]

The test was carried out in the same manner as in Test Example 3 except that a needle-equipped injector with a 30G needle (Dendronics needle 30G available from Misawa Medical Industry Co., Ltd.) was used as the injector.

The test was conducted also in a case of no treatment (in a case where the liquid pharmaceutical composition was not injected), in a case where the injector (needleless injector) illustrated in FIG. 1 was used as the injector and the same liquid pharmaceutical composition as prepared in Comparative Test Example 2 (Mg²⁺ concentration: 0) was used as the liquid pharmaceutical composition, and in a case where a needle-equipped injector with a 30G needle (Dendronics needle 30G available from Misawa Medical Industry Co., Ltd.) was used as the injector and the same liquid pharmaceutical composition as prepared in Comparative Test Example 2 (Mg²⁺ concentration: 0) was used as the liquid pharmaceutical composition.

The results are shown in Table 1. "Relative luminous intensity" in the table is a value obtained by performing conversion on the assumption that the luminous intensity in a case where the same liquid pharmaceutical composition as that prepared in Comparative Test Example 2 (Mg²⁺ concentration: 0) was used as the liquid pharmaceutical composition was 1.00 in each of Test Example 3 and Comparative Test Example 3.

Comparison between the conditions of Test Example 3 suggested that, for example, the luminous intensity in a case where the Mg²⁺ concentration was 0.0406 mM or more and less than 81.1 mM was higher than the luminous intensity in a case where the Mg²⁺ concentration was 0.

In addition, the gene expression increased when the Mg²⁺ concentration was in a range of 0.0406 mM to 40.6 mM (MgSO₄·7H₂O: a range of 0.01 mg/ml to 10 mg/ml). As shown from the results of the relative luminous intensity (which may be referred to as gene expression increase rate), the relative luminous intensity was only about 1.6 times at the maximum in Comparative Test Example 3, whereas the relative luminous intensity was about 7.4 times at the maximum in Test Example 3. The results revealed that the gene expression enhancing effect in a case where Mg²⁺ is present in the nucleic acid solution is a more remarkable phenomenon in a case where the injector (needleless injector) illustrated in FIG. 1 is used.

As shown from the results of the relative luminous intensity, in Test Example 3, the gene expression increase rate was a numerical value exceeding 1 in a range of the Mg²⁺ concentration from 0.0406 mM to 40.6 mM (MgSO₄·7H₂O: a range of 0.01 mg/ml to 10 mg/ml). Also in comparison with the corresponding gene expression increase rate of Comparative Test Example 3, the gene expression increase rate was a larger value in Test Example 3. Thus, it was revealed that the gene expression enhancing effect in a case where Mg²⁺ is present in the nucleic acid solution is particularly achieved when the Mg²⁺ concentration is in the range from 0.0406 mM to 40.6 mM (MgSO₄·7H₂O: a range of 0.01 mg/ml to 10 mg/ml).

**[Table 1]**

| Table 1 | | | | Magnesium sulfate | | | | | | | |
|---|---|---|---|---|---|---|---|---|---|---|---|
| | | No treatment | Mg²⁺ concentration 0 | 0.00406 mM (0.001 mg/ml) | 0.0406 mM (0.01 mg/ml) | 0.203 mM (0.05 mg/ml) | 0.406 mM (0.1 mg/ml) | 4.06 mM (1 mg/ml) | 40.6 mM (10 mg/ml) | 81.1 mM (20 mg/ml) | 203 mM (50 mg/ml) |
| Test Example 3 | Luminous intensity (A.U.) | 0 | 2794 | 2502 | 10480 | 11466 | 17906 | 20697 | 14992 | 676 | 0 |
| | Relative luminous intensity | | 1.00 | 0.90 | 3.75 | 4.10 | 6.41 | 7.41 | 5.37 | 0.24 | 0.00 |
| Comparative Test Example 3 | Luminous intensity (A.U.) | 0 | 16 | 18 | 7 | 11 | 26 | 23 | 26 | 23 | 10 |
| | Relative luminous intensity | | 1.00 | 1.13 | 0.44 | 0.67 | 1.63 | 1.44 | 1.65 | 1.43 | 0.61 |

### [Test Example 4]

The experiment was performed in the same manner as in Test Example 3 except that 11- to 13-week-old male balb/c mice (CLEA Japan, Inc.) were used as test animals, the final concentration of Mg²⁺ in the liquid pharmaceutical composition of Test Example 3 was 0.406 mM (MgSO₄·7H₂O was 0.1 mg/ml), and the same mice were euthanized 6 hours, 1 day, 2 days, 3 days, 5 days, 7 days, or 14 days after injection.

### [Comparative Test Example 4-1]

The same procedure as in Test Example 4 was repeated except that the same liquid pharmaceutical composition as that prepared in Comparative Test Example 2 (Mg²⁺ concentration: 0) was used.

### [Comparative Test Example 4-2]

The test was carried out in the same manner as in Test Example 4 except that a needle-equipped injector with a 30G needle (Dendronics needle 30G available from Misawa Medical Industry Co., Ltd.) was used as the injector.

### [Comparative Test Example 4-3]

The same procedure as in Test Example 4 was repeated except that a needle-equipped injector with a 30G needle (Dendronics needle 30G available from Misawa Medical Industry Co., Ltd.) was used as the injector and that the same liquid pharmaceutical composition as that prepared in Comparative Test Example 2 (Mg²⁺ concentration: 0) was used as the liquid pharmaceutical composition.

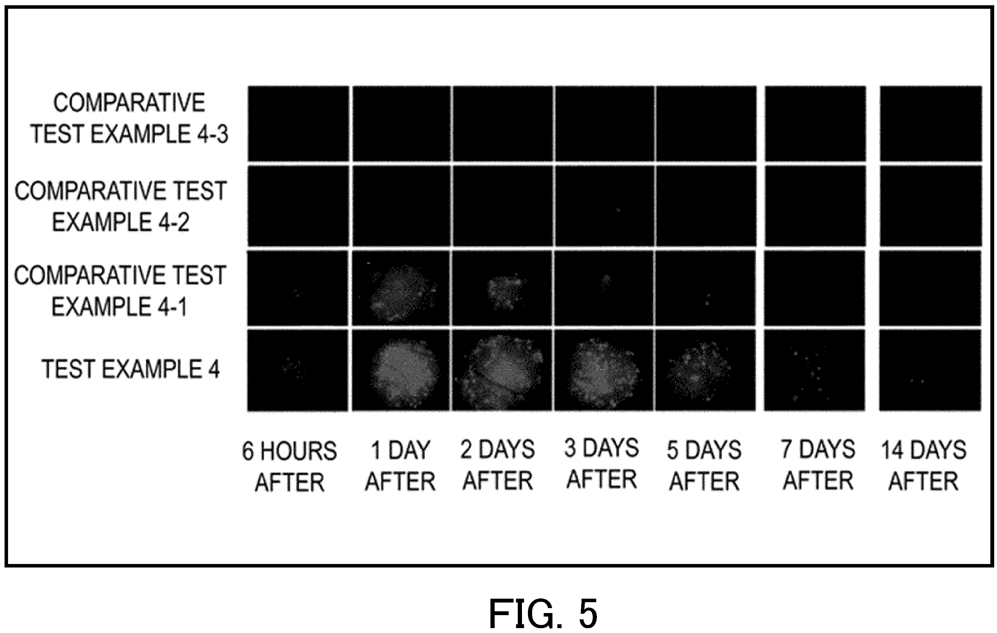

The results are shown in Table 2 and FIG. 5. "Relative luminous intensity (1)" in the table presents a value obtained by converting the luminous intensity of Test Example 4 based on the luminous intensity of Comparative Test Example 4-1, and "Relative luminous intensity (2)" presents a value obtained by converting the luminous intensity of Comparative Test Example 4-2 based on the luminous intensity of Comparative Test Example 4-3.

As shown from the results of Test Example 4, from 6 hours to 14 days after the injection of the liquid pharmaceutical composition, the luminous intensity in each case was higher than the luminous intensity in the case where the Mg²⁺ concentration was 0 (Comparative Test Example 4-1).

In Comparative Test Example 4-2 and Comparative Test Example 4-3, only very little fluorescence of GFP was observed from 6 hours to 14 days after the injection of the liquid pharmaceutical composition, indicating weak gene expression. In Comparative Test Example 4-1, strong fluorescence was observed 1 day after the injection, and slight fluorescence was observed 3 days after the injection. In contrast, in Test Example 4, strong fluorescence was observed 1 to 5 days after the injection, and it became difficult to observe fluorescence 14 days after the injection. These results revealed that the period of gene expression in the case of using the injector (needleless injector) illustrated in FIG. 1 is greatly extended by the presence of Mg²⁺ in the nucleic acid solution.

In addition, as shown from the results of the relative luminous intensity (1) and the relative luminous intensity (2) (which may be referred to as gene expression increase rate), all values exceeded 1 from 6 hours to 14 days after the injection of the liquid pharmaceutical composition. Furthermore, the relative luminous intensity (1) was larger than the relative luminous intensity (2) at any time from 6 hours to 14 days after the injection of the liquid pharmaceutical composition. Thus, the gene expression enhancing effect was confirmed in a case where Mg²⁺ was present in the nucleic acid solution at any time from 6 hours to 14 days after the injection of the liquid pharmaceutical composition. In particular, in a case where the injector (needleless injector) illustrated in FIG. 1 was used and Mg²⁺ was present in the nucleic acid solution, the gene expression increase rate on 2 days and 3 days after the injection of the liquid pharmaceutical composition exceeded 60, indicating greatly improved gene expression.

### [Table 2]

**Table 2**

| | 6 hours after | 1 day after | 2 days after | 3 days after | 5 days after | 7 days after | 14 days after |
|---|---|---|---|---|---|---|---|
| Comparative Test Example 4-1 | 101 | 2794 | 438 | 384 | 246 | 112 | 109 |
| Test Example 4 | 401 | 17906 | 26351 | 24313 | 9797 | 1071 | 189 |
| Relative Luminous intensity (1) | 3.96 | 6.41 | 60.16 | 63.38 | 39.81 | 9.58 | 1.74 |
| Comparative Test Example 4-3 | 0 | 7.5 | 0.2 | 0.4 | 0.2 | 0.2 | 0.2 |
| Comparative Test Example 4-2 | 0 | 7.7 | 1.0 | 0.7 | 0.4 | 0.3 | 0.2 |
| Relative Luminous intensity (2) | 1.1 | 1.0 | 4.1 | 1.7 | 1.6 | 1.9 | 1.3 |

### [Test Example 5]

PBS-tablet (available from Takara Bio Inc.) (PBS(-)) was dissolved in water for injection (available from Otsuka Pharmaceutical Co., Ltd.) to prepare 2-fold PBS, which was sterilized with a filter. Magnesium chloride (MgCl₂·6H₂O, Nacalai Tesque), magnesium nitrate (Mg(NO₃)₂·6H₂O, Nacalai Tesque), or magnesium aspartate (Angene International Limited, Inc.) was dissolved in the prepared 2-fold PBS to achieve a concentration twice as high as that when injected into an 11-week-old male balb/c mouse (CLEA Japan Inc.) as a test animal, followed by filter sterilization. Thereafter, the resultant solution was mixed with CMV-DASHER-GFP (available from ATUM) (solvent: TE) (1.0 mg/mL), which was a plasmid DNA containing a GFP gene, at a ratio of 1:1 to prepare a liquid pharmaceutical composition. Specifically, liquid pharmaceutical compositions were prepared in such a manner that the final concentration of the plasmid DNA containing a GFP gene was 0.5 mg/mL, the final Mg²⁺ concentration was 4.92 mM (final concentration of MgCl₂·6H₂O: 1 mg/ml) for magnesium chloride (MgCl₂·6H₂O), the final Mg²⁺ concentration was 3.90 mM (final concentration of Mg(NO₃)₂·6H₂O: 1 mg/ml) for magnesium nitrate (Mg(NO₃)₂·6H₂O), and the final Mg²⁺ concentration was 3.47 mM for magnesium aspartate (final concentration of magnesium aspartate: 1 mg/ml).

In addition to the injection into mice, acquisition of bright field images and fluorescent images, acquisition of superimposed images, and analysis of gene expression were performed in the same manner as in Test Example 2.

### [Comparative Test Example 5]

The test was carried out in the same manner as in Test Example 5 except that a needle-equipped injector with a 30G needle (Dendronics needle 30G available from Misawa Medical Industry Co., Ltd.) was used as the injector.

The test was also carried out in a case where the injector (needleless injector) illustrated in FIG. 1 was used as the injector and the same liquid pharmaceutical composition as that prepared in Comparative Test Example 2 (Mg²⁺ concentration: 0) was used as the liquid pharmaceutical composition, and in a case where the needle-equipped injector with a 30G needle (Dendronics needle 30G available from Misawa Medical Industry Co., Ltd.) was used as the injector and the same liquid pharmaceutical composition as that prepared in Comparative Test Example 2 (Mg²⁺ concentration: 0) was used as the liquid pharmaceutical composition.

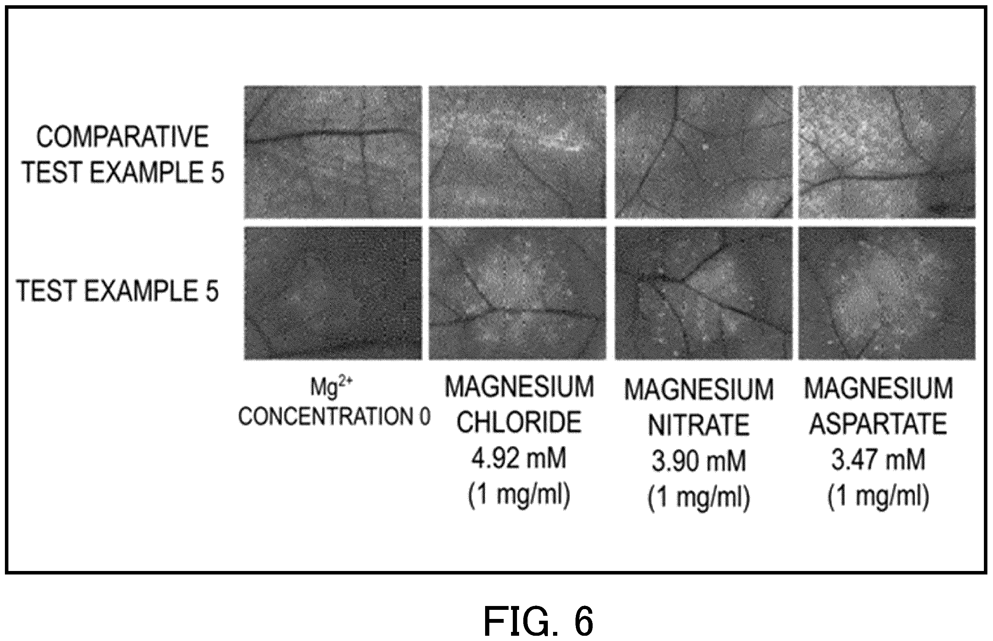

The results are shown in Table 3 and FIG. 6. "Relative luminous intensity" in the table is a value obtained by performing conversion on the assumption that the luminous intensity in the case where the same liquid pharmaceutical composition as that prepared in Comparative Test Example 2 (Mg²⁺ concentration: 0) was used as the liquid pharmaceutical composition was 1.00 in each of Test Example 5 and Comparative Test Example 5.

As shown from the results of Test Example 5, the luminous intensity in a case where any of the magnesium salts was used was higher than the luminous intensity in the case where the Mg²⁺ concentration was 0.

In addition, in the case where any of the magnesium salts was used, gene expression was increased as compared with the case where the Mg²⁺ concentration was 0. As shown from the results of the relative luminous intensity (which may be referred to as gene expression increase rate), in Test Example 5, a value exceeding 1 was obtained in the case where any of the magnesium salts was used. Also in comparison with the corresponding gene expression increase rate of Comparative Test Example 5, a large value was shown in Test Example 5. Thus, it was revealed that the gene expression enhancing effect in the case where Mg²⁺ is present in the nucleic acid solution can be obtained regardless of the type of the magnesium salt.

### [Table 3]

**Table 3**

| | | Mg²⁺ concentration 0 | Magnesium chloride 4.92 mM (1 mg/ml) | Magnesium nitrate 3.90 mM (1 mg/ml) | Magnesium aspartate 3.47 mM (1 mg/ml) |
|---|---|---|---|---|---|
| Test Example 5 | Luminous intensity (A.U.) | 3538.59 | 24660.87 | 17803.56 | 35038.57 |
| | Relative luminous intensity | 1.00 | 6.97 | 5.03 | 9.90 |
| Comparative Test Example 5 | Luminous intensity (A.U.) | 16.30 | 21.67 | 30.90 | 10.50 |
| | Relative luminous intensity | 1.00 | 1.33 | 1.90 | 0.64 |

### [Test Example 6]

PBS-tablet (available from Takara Bio Inc.) (PBS(-)) was dissolved in water for injection (available from Otsuka Pharmaceutical Co., Ltd.) to prepare 2-fold PBS, which was sterilized with a filter. Calcium chloride (CaCl₂·2H₂O, Nacalai Tesque), manganese(II) sulfate (MnSO₄·5H₂O, Nacalai Tesque), or lithium chloride (LiCl, Nacalai Tesque) was dissolved in the prepared 2-fold PBS to achieve a concentration twice as high as that when injected into an 11-week-old male balb/c mouse (CLEA Japan Inc.) as a test animal, followed by filter sterilization. Thereafter, the resultant solution was mixed with CMV-DASHER-GFP (available from ATUM) (solvent: TE) (1.0 mg/mL), which was a plasmid DNA containing a GFP gene, at a ratio of 1:1 to prepare a liquid pharmaceutical composition. Specifically, liquid pharmaceutical compositions were prepared in such a manner that the final concentration of the plasmid DNA containing a GFP gene was 0.5 mg/mL, the final Ca²⁺ concentration was 6.8 mM for calcium chloride (CaCl₂·2H₂O) (final concentration of CaCl₂·2H₂O: 1 mg/ml), the final Mn²⁺ concentration was 4.15 mM for manganese(II) sulfate (MnSO₄·5H₂O) (final concentration of MnSO₄·5H₂O: 1 mg/ml), and the final Li⁺ concentration was 23.5 mM (final concentration of LiCl: 1 mg/ml) for lithium chloride (LiCl).

In addition to the injection into mice, acquisition of bright field images and fluorescent images, acquisition of superimposed images, and analysis of gene expression were performed in the same manner as in Test Example 2.

### [Comparative Test Example 6]

The test was carried out in the same manner as in Test Example 6 except that a needle-equipped injector with a 30G needle (Dendronics needle 30G available from Misawa Medical Industry Co., Ltd.) was used as the injector.

The test was also carried out in a case where the injector (needleless injector) illustrated in FIG. 1 was used as the injector and the same liquid pharmaceutical composition as that prepared in Comparative Test Example 2 (Ca²⁺ concentration, Mn²⁺ concentration, and Li⁺ concentration: 0) was used as the liquid pharmaceutical composition, and in a case where a needle-equipped injector with a 30G needle (Dendronics needle 30G available from Misawa Medical Industry Co., Ltd.) was used as the injector and the same liquid pharmaceutical composition as prepared in Comparative Test Example 2 (Ca²⁺ concentration, Mn²⁺ concentration, and Li⁺ concentration: 0) was used as the liquid pharmaceutical composition.

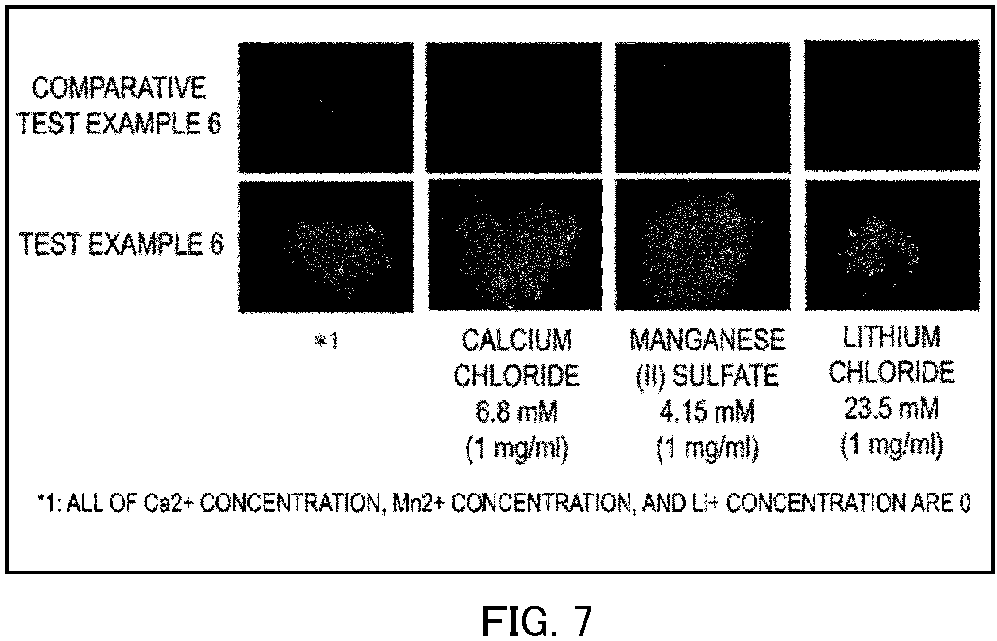

The results are shown in Table 4 and FIG. 7. "Relative luminous intensity" in the table is a value obtained by performing conversion on the assumption that the luminous intensity in a case where the same liquid pharmaceutical composition as that prepared in Comparative Test Example 2 (Ca²⁺ concentration, Mn²⁺ concentration, and Li⁺ concentration: 0) was used as the liquid pharmaceutical composition was 1.00 in each of Test Example 6 and Comparative Test Example 6.

As shown from the comparison between the conditions of Test Example 6, both the luminous intensities in the case where calcium chloride was used and in the case where manganese(II) sulfate was used were higher than the luminous intensity in the case where both the concentrations were 0.

As shown from the results of the relative luminous intensity (which may be referred to as gene expression increase rate), in Test Example 6, the values exceeded 1 in the case of using calcium chloride or manganese(II) sulfate. Also in comparison with the corresponding gene expression increase rate of Comparative Test Example 6, a large value was shown in Test Example 6. Thus, in a case where a divalent cation was present in the nucleic acid solution, the gene expression enhancing effect was obtained, whereas in a case where a monovalent Li⁺ was present in the nucleic acid solution, the gene expression increase rate was less than 1, and the gene expression enhancing effect was not obtained. These results were in good agreement with the results of DLS (Test Example 1, Comparative Test Example 1-1, Comparative Test Example 1-2, and Comparative Test Example 1-3). The results supported that the gene expression enhancing effect in the case of using the injector (needleless injector) illustrated in FIG. 1 is a phenomenon peculiar to the case where the divalent cation is present in the nucleic acid solution and is caused by the particle formation of the nucleic acid.

### [Table 4]

**Table 4**

| | | *1 | Calcium chloride 6.8 mM (1 mg/ml) | Manganese (II) sulfate 4.15 mM (1 mg/ml) | Lithium chloride 23.5 mM (1 mg/ml) |
|---|---|---|---|---|---|
| Test Example 6 | Luminous intensity (A.U.) | 4619.75 | 8686.77 | 9989.20 | 4171.30 |
| | Relative luminous intensity | 1.00 | 1.88 | 2.16 | 0.90 |
| Comparative Test Example 6 | Luminous intensity (A.U.) | 8.06 | 6.45 | 3.54 | 1.51 |
| | Relative luminous intensity | 1.00 | 0.80 | 0.44 | 0.19 |

| | | | | | |
|---|---|---|---|---|---|
| *1 Ca²⁺ concentration, Mn²⁺ concentration, and Li⁺ concentration are all 0 | | | | | |

### [Test Example 7]

The particle formation of nucleic acid in the case where Mg²⁺ was present in the nucleic acid solution was observed directly by AFM. Measurement by AFM was carried out using AFM5000/AFM5300E available from Hitachi High-Tech Corporation. SI-DF20S (available from Olympus Corporation) for biological samples was used as a cantilever.

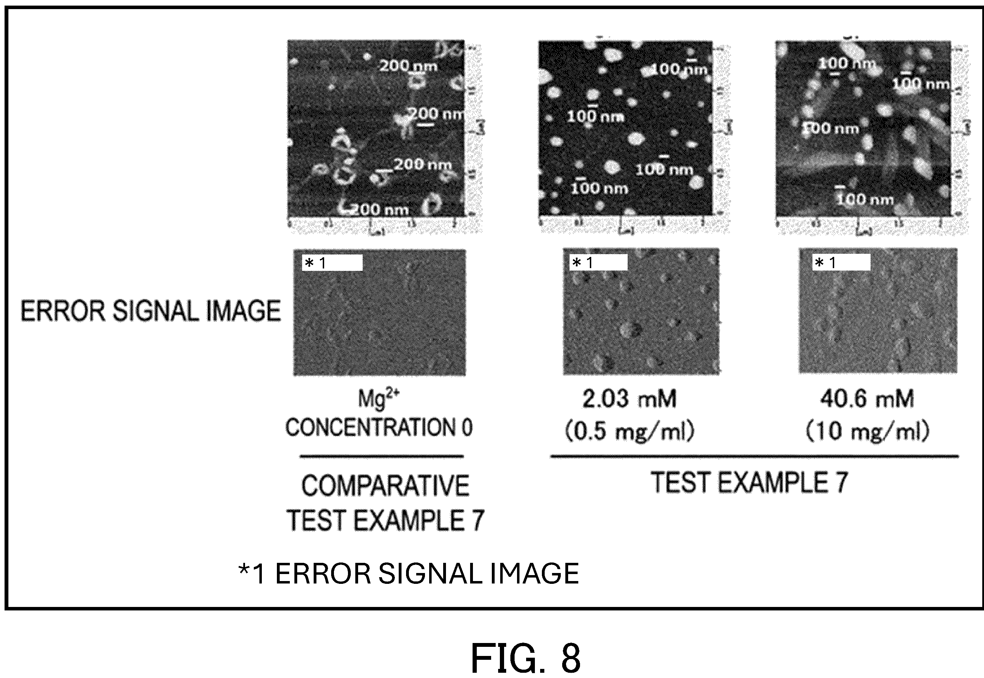

PBS-tablet (available from Takara Bio Inc.) (PBS(-)) was dissolved in water for injection (available from Otsuka Pharmaceutical Co., Ltd.) to prepare 2-fold PBS, which was sterilized with a filter. Magnesium sulfate (MgSO₄·7H₂O) (Nacalai Tesque) was dissolved in the prepared 2-fold PBS to achieve each of the final concentrations shown in FIG. 8, followed by filter sterilization. Thereafter, the resultant solution was mixed with CMV-DASHER-GFP (available from ATUM) (solvent: TE) (1.0 mg/mL), which was a plasmid DNA containing a GFP gene, at a ratio of 1:1 to prepare a liquid pharmaceutical composition. Specifically, the liquid pharmaceutical compositions were prepared in such a manner that the final concentration of the plasmid DNA containing a GFP gene was 0.5 mg/mL and the final Mg²⁺ concentration (the final concentration of MgSO₄·7H₂O is shown in parentheses) was 2.03 mM (0.5 mg/ml) or 40.6 mM (10 mg/ml). Each of the prepared liquid pharmaceutical compositions was added dropwise onto a fresh mica surface, adsorbed for a predetermined time, and dried by air blow for use.

### [Comparative Test Example 7]

PBS-tablet (available from Takara Bio Inc.) (PBS(-)) was dissolved in water for injection (available from Otsuka Pharmaceutical Co., Ltd.) to prepare 2-fold PBS, which was sterilized with a filter. Thereafter, the resultant solution was mixed with CMV-DASHER-GFP (available from ATUM) (solvent: TE) (1.0 mg/mL), which was a plasmid DNA containing a GFP gene, at a ratio of 1:1 to prepare a liquid pharmaceutical composition. Specifically, a liquid pharmaceutical composition was prepared in which the final concentration of the plasmid DNA containing a GFP gene was 0.5 mg/mL and the final concentration of Mg²⁺ was 0. The other conditions were the same as in Test Example 7.

The results are shown in FIG. 8.

In Comparative Test Example 7, a circular structure of about 200 nm was confirmed. In contrast, particles of about 100 nm were observed in Test Example 7. These results were in good agreement with the results of DLS (Test Example 1, Comparative Test Example 1-1, Comparative Test Example 1-2, and Comparative Test Example 1-3), and it was confirmed that in the case where Mg²⁺ is present in the nucleic acid solution, the nucleic acid is certainly formed into particles. The results supported that the gene expression enhancing effect in the case where Mg²⁺ is present in the nucleic acid solution and the injector (needleless injector) illustrated in FIG. 1 is used is caused by the particle formation of the nucleic acid.

### [Test Example 8]

Liquid pharmaceutical compositions were prepared in such a manner that in Test Example 2, a spring-type needleless injector (BEAUTTO Co., Ltd., hyaluronic acid syringe, Amazon Standard Identification Number (ASIN): B08NCHTRHZ) was used instead of the injector (needleless injector) illustrated in FIG. 1, magnesium sulfate (MgSO₄·7H₂O, Nacalai Tesque) was used instead of magnesium chloride (MgCl₂·6H₂O), the final concentration of the plasmid DNA containing a GFP gene was 0.5 mg/mL, and the Mg²⁺final concentration (the final concentration of MgSO₄·7H₂O is shown in parentheses) was 2.03 mM (0.5 mg/ml) or 40.6 mM (10 mg/ml).

In addition to the injection into mice, acquisition of bright field images and fluorescent images, acquisition of superimposed images, and analysis of gene expression were performed in the same manner as in Test Example 2.

### [Comparative Test Example 8]

The test was carried out in the same manner as in Test Example 8 except that a needle-equipped injector with a 30G needle (Dendronics needle 30G available from Misawa Medical Industry Co., Ltd.) was used as the injector.

The test was also carried out in a case where the spring-type needleless injector was used as the injector and the same liquid pharmaceutical composition as that prepared in Comparative Test Example 2 (Mg²⁺ concentration: 0) was used as the liquid pharmaceutical composition, and in a case where a needle-equipped injector with a 30G needle (Dendronics needle 30G available from Misawa Medical Industry Co., Ltd.) was used as the injector and the same liquid pharmaceutical composition as that prepared in Comparative Test Example 2 (Mg²⁺ concentration: 0) was used as the liquid pharmaceutical composition.

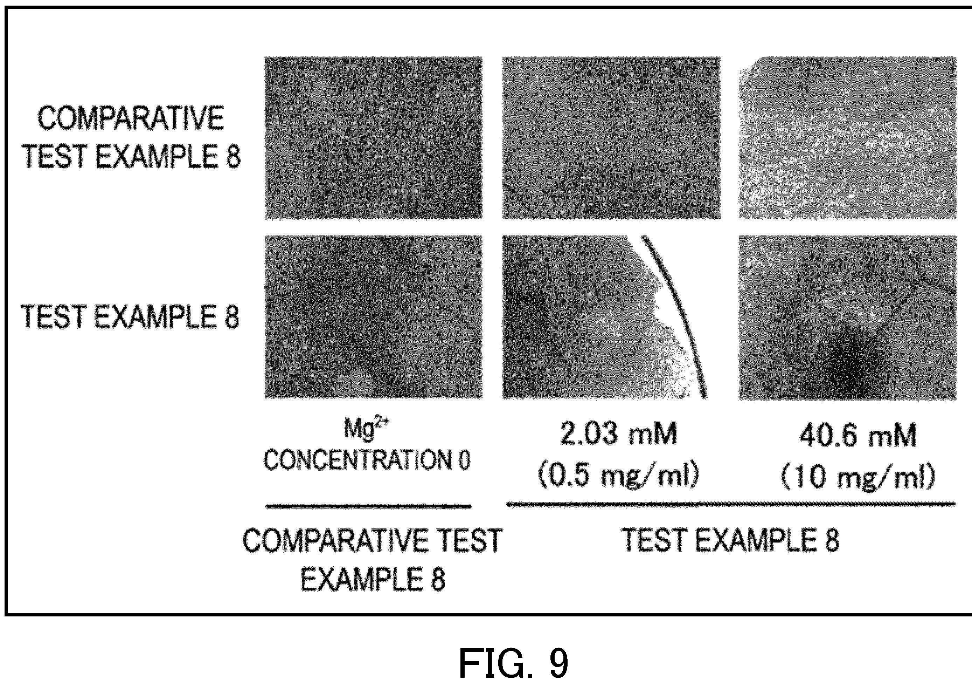

The results are shown in Table 5 and FIG. 9. "Relative luminous intensity" in the table is a value obtained by performing conversion on the assumption that the luminous intensity in the case where the same liquid pharmaceutical composition as that prepared in Comparative Test Example 2 (Mg²⁺ concentration: 0) was used as the liquid pharmaceutical composition was 1.00 in each of Test Example 8 and Comparative Test Example 8.

As shown from the results of Test Example 8, the luminous intensity in the case where Mg²⁺ was present in the nucleic acid solution was higher than the luminous intensity in the case where the Mg²⁺ concentration was 0.

In Test Example 8, the optimum conditions as ejection conditions of the spring-type needleless injector were not examined, and thus gene expression remained low in all cases. However, as shown from the results of the relative luminous intensity (which may be referred to as gene expression increase rate), all values exceeded 1 in the case where Mg²⁺ was present in the nucleic acid solution. Also in comparison with the corresponding gene expression increase rate of Comparative Test Example 8, a large value was shown in Test Example 8. Accordingly, it was shown that the gene expression enhancing effect in the case where Mg²⁺ is present in the nucleic acid solution is not limited to the injector using the combustion energy of the ignition agent as the ejection energy, but can also be obtained by the spring-type needleless injector.

### [Table 5]

**Table 5**

| | | Mg²⁺ Concentration 0 | Magnesium sulfate 2.03 mM (0.5 mg/ml) | Magnesium sulfate 40.6 mM (10 mg/ml) |
|---|---|---|---|---|
| Test Example 8 | Luminous intensity (A.U.) | 98.41 | 1047.94 | 1037.73 |
| | Relative luminous intensity | 1.00 | 10.65 | 10.55 |
| Comparative Test Example 8 | Luminous intensity (A.U.) | 16.06 | 8.99 | 26.46 |
| | Relative luminous intensity | 1.00 | 0.56 | 1.65 |

### [Test Example 9]

PBS-tablet (available from Takara Bio Inc.) (PBS(-)) was dissolved in water for injection (available from Otsuka Pharmaceutical Co., Ltd.) to prepare 2-fold PBS, which was sterilized with a filter. Magnesium sulfate (MgSO₄·7H₂O, Nacalai Tesque) was dissolved in the prepared 2-fold PBS to achieve a concentration twice as high as that when injected into an 11-week-old male balb/c mouse (CLEA Japan, Inc.) as a test animal, followed by filter sterilization. Then, the sterilized solution was mixed with mRNA (unmodified) encoding GFP as a reporter protein (available from OZ Biosciences, Funakoshi Cat#. MRNA15-100) (solvent: TE) (0.2 mg/mL) at a ratio of 1:1 to prepare a liquid pharmaceutical composition. Specifically, liquid pharmaceutical compositions were prepared in such a manner that the final concentration of the mRNA encoding GFP was 0.1 mg/mL (2.0 µg per injection) and the final concentration of Mg²⁺ (the final concentration of is shown in parentheses) was 2.03 mM (0.5 mg/ml), 4.06 mM (1 mg/ml), or 40.6 mM (10 mg/ml).

In addition to the injection into mice, acquisition of bright field images and fluorescent images, acquisition of superimposed images, and analysis of gene expression were performed in the same manner as in Test Example 2.

### [Comparative Test Example 9]

The test was carried out in the same manner as in Test Example 9 except that a needle-equipped injector with a 30G needle (Dendronics needle 30G available from Misawa Medical Industry Co., Ltd.) was used as the injector.

The test was also carried out in a case where the injector (needleless injector) illustrated in FIG. 1 was used as the injector and the same liquid pharmaceutical composition as that prepared in Comparative Test Example 2 (Mg²⁺ concentration: 0) was used as the liquid pharmaceutical composition, and in a case where the needle-equipped injector with a 30G needle (Dendronics needle 30G available from Misawa Medical Industry Co., Ltd.) was used as the injector and the same liquid pharmaceutical composition as that prepared in Comparative Test Example 2 (Mg²⁺ concentration: 0) was used as the liquid pharmaceutical composition.

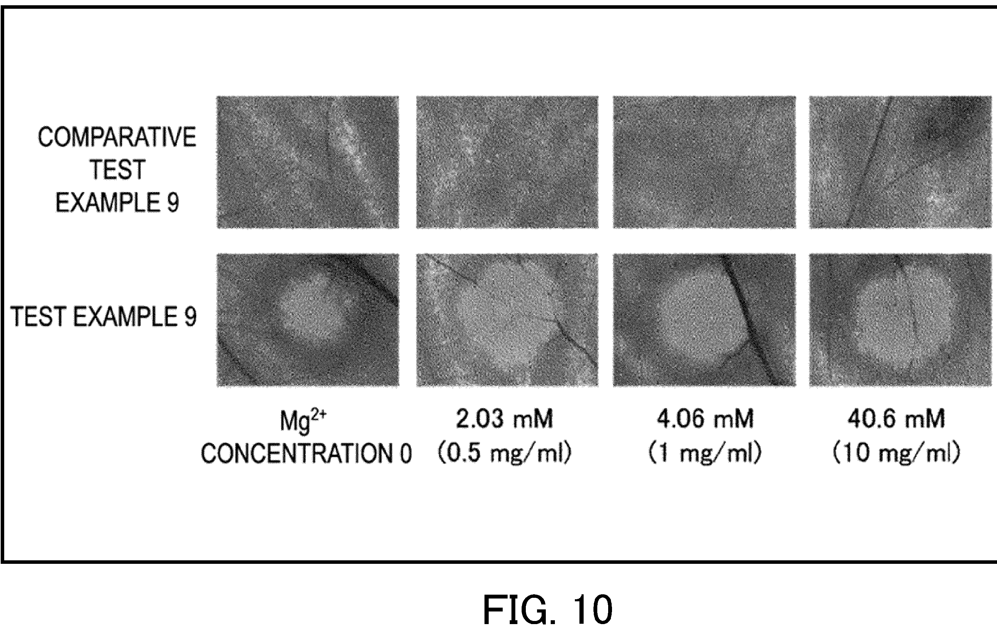

The results are shown in Table 6 and FIG. 10. "Relative luminous intensity" in the table is a value obtained by performing conversion on the assumption that the luminous intensity in the case where the same liquid pharmaceutical composition as that prepared in Comparative Test Example 2 (Mg²⁺ concentration: 0) was used as the liquid pharmaceutical composition was 1.00 in each of Test Example 9 and Comparative Test Example 9.

As shown from the comparison between the conditions of Test Example 9, the luminous intensity in any case where Mg²⁺ was present in the nucleic acid solution was greater than the luminous intensity in the case where the Mg²⁺ concentration was 0.

In Test Example 9, in any case where Mg²⁺ was present in the nucleic acid solution, strong GFP fluorescence was observed. As shown from the results of the relative luminous intensity (which may be referred to as gene expression increase rate), in Test Example 9, in the case where Mg²⁺ was present in the nucleic acid solution, all values exceeded 1. Also in comparison with the corresponding gene expression increase rate of Comparative Test Example 9, a large value was shown in Test Example 9. The results indicated that the gene expression enhancing effect in the case where Mg²⁺ is present in the nucleic acid solution and the injector (needleless injector) illustrated in FIG. 1 is used is obtained in a nucleic acid in a broad sense including mRNA.

### [Table 6]

**Table 6**

| | | Mg²⁺ concentration 0 | Magnesium sulfate 2.03 mM (0.5 mg/ml) | Magnesium sulfate 4.06 mM (1 mg/ml) | Magnesium sulfate 40.6 mM (10 mg/ml) |
|---|---|---|---|---|---|
| Test Example 9 | Luminous intensity (A.U.) | 14439.91 | 33484.08 | 31662.24 | 23686.27 |
| | Relative luminous intensity | 1.00 | 2.32 | 2.19 | 1.64 |
| Comparative Test Example 9 | Luminous intensity (A.U.) | 25.95 | 30.92 | 34.48 | 20.75 |
| | Relative luminous intensity | 1.00 | 1.19 | 1.33 | 0.80 |

### [Test Example 10]

The experiment was performed in the same manner as in Test Example 9 except that 11- to 13-week-old male balb/c mice (CLEA Japan, Inc.) were used as test animals, the final concentration of Mg²⁺ in the liquid pharmaceutical composition of Test Example 9 was 2.03 mM (MgSO₄·7H₂O was 0.5 mg/ml), and the mice were euthanized 6 hours, 1 day, 2 days, 3 days, 5 days, 7 days, or 14 days after the injection.

### [Comparative Test Example 10-1]

The same procedure as in Test Example 10 was repeated except that the same liquid pharmaceutical composition as that prepared in Comparative Test Example 2 (Mg²⁺ concentration: 0) was used as the liquid pharmaceutical composition.

### [Comparative Test Example 10-2]

The same procedure as in Test Example 10 was repeated except that a needle-equipped injector with a 30G needle (Dendronics needle 30G available from Misawa Medical Industry Co., Ltd.) was used as the injector.

### [Comparative Test Example 10-3]

The same procedure as in Test Example 10 was repeated except that a needle-equipped injector with a 30G needle (Dendronics needle 30G available from Misawa Medical Industry Co., Ltd.) was used as the injector and that the same liquid pharmaceutical composition as that prepared in Comparative Test Example 2 (Mg²⁺ concentration: 0) was used as the liquid pharmaceutical composition.

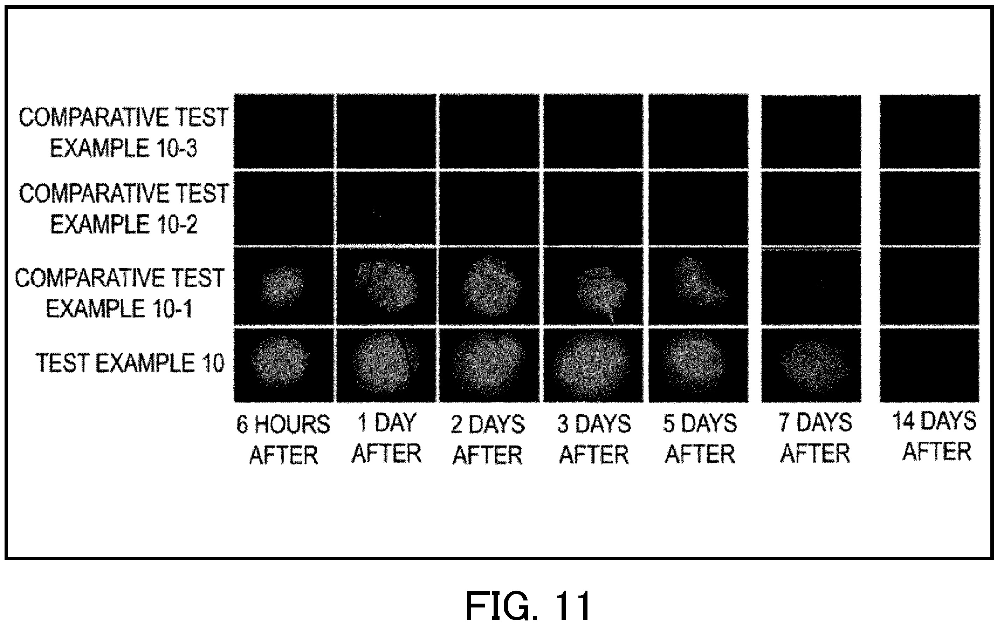

The results are shown in Table 7 and FIG. 11. "Relative luminous intensity (1)" in the table is a value obtained by converting the luminous intensity of Test Example 10 based on the luminous intensity of Comparative Test Example 10-1, and "Relative luminous intensity (2)" is a value obtained by converting the luminous intensity of Comparative Test Example 10-2 based on the luminous intensity of Comparative Test Example 10-3.

As shown from the results of Test Example 10, the luminous intensity in any case from 6 hours to 14 days after the injection of the liquid pharmaceutical composition was higher than the luminous intensity in the case where the Mg²⁺ concentration was 0 (Comparative Test Example 10-1).

In Comparative Test Example 10-1, strong fluorescence was observed from 6 hours after the injection, and slight fluorescence was observed 7 days after the injection. In contrast, in Test Example 10, strong fluorescence was observed from 6 hours to 7 days after the injection, and weak fluorescence was observed 14 days after the injection.

As shown from the results of the relative luminous intensity (1) (which may be referred to as gene expression increase rate), the relative luminous intensity (1) exceeded 1 in any case from 6 hours to 14 days after the injection of the liquid pharmaceutical composition. Furthermore, the relative luminous intensity (1) was larger than the relative luminous intensity (2) at any time from 6 hours to 14 days after the injection of the liquid pharmaceutical composition. Thus, even when mRNA was used as the nucleic acid, the gene expression enhancing effect in the case where Mg²⁺ was present in the nucleic acid solution was confirmed from 6 hours to 14 days after the injection of the liquid pharmaceutical composition using the injector (needleless injector) illustrated in FIG. 1.

### [Table 7]

**Table 7**

| | 6 hours after | 1 day after | 2 days after | 3 days after | 5 days after | 7 days after | 14 days after |
|---|---|---|---|---|---|---|---|
| Comparative Test Example 10-1 | 18476 | 14440 | 16547 | 7336 | 6703 | 393 | 1 |
| Test Example 10 | 33486 | 35294 | 33438 | 27438 | 25501 | 16855 | 3 |
| Relative Luminous intensity (1) | 1.81 | 2.44 | 2.02 | 3.74 | 3.80 | 42.88 | 4.12 |
| Comparative Test Example 10-3 | 0.04 | 6.97 | 0.50 | 0.11 | 1.40 | 0.84 | 0.40 |
| Comparative Test Example 10-2 | 0.05 | 12.38 | 0.60 | 0.10 | 0.53 | 0.77 | 0.29 |
| Relative Luminous intensity (2) | 1.20 | 1.78 | 1.20 | 0.90 | 0.38 | 0.92 | 0.73 |

### [Test Example 11]

### (Preparation of Liquid Pharmaceutical Composition)

PBS-tablet (available from Takara Bio Inc.) (PBS(-)) was dissolved in water for injection (available from Otsuka Pharmaceutical Co., Ltd.) to prepare 2-fold PBS, which was sterilized with a filter. Magnesium sulfate (MgSO₄·7H₂O, Nacalai Tesque) was dissolved in the prepared 2-fold PBS to achieve a concentration twice as high as that when injected into an 11-week-old male C57BL6 mouse (CLEA Japan, Inc.) as a test animal, followed by filter sterilization. Then, the sterilized solution was mixed with mRNA encoding ovalbumin (OVA) (available from OZ Biosciences, Funakoshi Cat#. MRNA42-100) (solvent: TE) (0.2 mg/mL) at a ratio of 1:1 to prepare a liquid pharmaceutical composition. Specifically, the liquid pharmaceutical composition was prepared in such a manner that the final concentration of the mRNA encoding OVA was 0.1 mg/mL (2.0 µg per injection) and the final Mg²⁺ concentration (the final concentration of is shown in parentheses) was 2.03 mM (0.5 mg/ml).

### (Injection into Mouse)

An 11-week-old male C57BL6 mouse was anesthetized by aspiration with isoflurane, and then its back was clipped using animal hair clippers. Thereafter, the back was disinfected with alcohol, and the liquid pharmaceutical composition was injected into two sites of the back using the following injector. 20 µL of the liquid pharmaceutical composition per site was injected. The injector used was the injector illustrated in FIG. 1 (needleless injector), with a nozzle diameter of 0.1 mm. 25 mg of ZPP was used as an ignition agent for the injector, and 40 mg of a single base smokeless explosive (containing 98 mass% of nitrocellulose, 0.8 mass% of diphenylamine, and 1.2 mass% of potassium sulfate) was used as a gas generating agent.

### (ELISpot Assay)

On Day 9 after the injection of the liquid pharmaceutical composition, the mouse was euthanized, and the spleen was isolated. A medium attached to an ELISpot kit (Mouse IFN-γ Single-Color ELISPOT (available from CTL)) was added to the isolated spleen, and then a cell suspension was prepared using a cell strainer. Thereafter, hemolysis and washing were performed using a VersaLyse hemolysis reagent (Beckman Coulter, Inc.) to prepare a cell dispersion of the spleen. Cells were counted using a disposable cell counter (C-Chip, Digital Bio).

Cells producing INF-γ in response to an epitope peptide of an MHC class I molecule of OVA (amino acid sequence: SIINFEKL (SEQ ID NO: 1), synthesis of which was entrusted to PH Japan, Co., Ltd.) were detected by an ELISpot assay (Mouse IFN-γ Single-Color ELISPOT (available from CTL)). Specifically, 100 µL of the cell dispersion was seeded on a plate pre-coated with a detection antibody to achieve a predetermined number of cells (5 × 10⁵ cells), and then the total amount per well was adjusted to 200 µL using a medium attached to the kit. In a case of stimulation with an antigen, a medium containing 40 µg/mL of the peptide (amino acid sequence: SIINFEKL (SEQ ID NO: 1)) as an antigen was used to adjust the total amount per well to 200 µL. The cells were then cultured overnight, and the plate was colored. The plate was colored in accordance with the protocol described in the kit.

### [Comparative Test Example 11-1]

PBS-tablet (available from Takara Bio Inc.) (PBS(-)) was dissolved in water for injection (available from Otsuka Pharmaceutical Co., Ltd.) to prepare 2-fold PBS, which was sterilized with a filter. Magnesium sulfate (MgSO₄·7H₂O, Nacalai Tesque) was dissolved in the prepared 2-fold PBS to achieve a concentration twice as high as that when injected into an 11-week-old male C57BL6 mouse (CLEA Japan, Inc.) as a test animal, followed by filter sterilization. Then, the sterilized solution was mixed with mRNA encoding ovalbumin (OVA) (available from OZ Biosciences, Funakoshi Cat#. MRNA42-100) (solvent: TE) (0.2 mg/mL) at a ratio of 1:1 to prepare a liquid pharmaceutical composition. Specifically, the final concentration of the mRNA encoding OVA was adjusted to 0.1 mg/mL (2.0 µg per injection) to prepare a liquid pharmaceutical composition having a Mg²⁺ concentration of 0. The other conditions were the same as in Test Example 11. In other words, in this comparative test example, a liquid pharmaceutical composition containing mRNA encoding OVA and having a Mg²⁺ concentration of 0 was injected using the injector (needleless injector) illustrated in FIG. 1.

### [Comparative Test Example 11-2]

The test was carried out in the same manner as in Comparative Test Example 11-1 except that a needle-equipped injector with a 30G needle (Dendronics needle 30G available from Misawa Medical Industry Co., Ltd.) was used as the injector. In other words, in this comparative test example, a liquid pharmaceutical composition containing mRNA encoding OVA and having a Mg²⁺ concentration of 0 was injected using a needle-equipped injector.

### [Comparative Test Example 11-3]

1-fold PBS was used as a liquid pharmaceutical composition. The other conditions were the same as in Test Example 11. In other words, in this comparative test example, 1-fold PBS serving as a liquid pharmaceutical composition was injected using the injector (needleless injector) illustrated in FIG. 1.

### [Comparative Test Example 11-4]

An untreated case (a case where the liquid pharmaceutical composition was not injected) was also prepared.

### [Comparative Test Example 11-5]

The test was carried out in the same manner as in Test Example 11 except that a needle-equipped injector with a 30G needle (Dendronics needle 30G available from Misawa Medical Industry Co., Ltd.) was used as the injector. In other words, in this comparative test example, a liquid pharmaceutical composition containing mRNA encoding OVA and having a final Mg²⁺ concentration (the final concentration of is shown in parentheses) of 2.03 mM (0.5 mg/ml) was injected using a needle-equipped injector.

### The results are shown in FIG. 12A, FIG. 12B, and FIG. 12C.

Firstly, as illustrated in FIG. 12A, in Comparative Test Example 11-2, Comparative Test Example 11-3, and Comparative Test Example 11-4, no spot was formed, and cellular immunity was not induced. In contrast, in Comparative Test Example 11-1, many spots were formed, and cellular immunity was induced.

Next, as illustrated in FIG. 12B, in Test Example 11, a significantly larger number of spots were formed than in Comparative Test Example 11-1, and induction of cellular immunity was improved. Thus, it is presumed that even in a case where the nucleic acid solution was injected in the same manner using the injector (needleless injector) illustrated in FIG. 1, the presence of Mg²⁺ in the nucleic acid solution increased the expression level of the protein in the individual mouse (living body) and enhanced cellular immunity. Meanwhile, as illustrated in FIG. 12C, in the case of needle injection, an increase in the number of spots was not confirmed even when Mg²⁺ was present in the nucleic acid solution.

The results revealed that the effect of the nucleic acid vaccine can be obtained when the injector (needleless injector) illustrated in FIG. 1 is used. The results also revealed that the presence of Mg²⁺ in the nucleic acid solution enhances the induction of cellular immunity to improve the effect of the nucleic acid vaccine.

### REFERENCE SIGNS LIST

1. Injector, 2. Housing, 3. Syringe section, 4. Plunger, 5. Piston, 6. Injector body, 7. Drive section, 8. Button, 9. Battery, 10. Injector assembly, 31. Nozzle section, 31a. Ejection port, 32. Storage section, 71. Igniter

## Claims

1. A liquid pharmaceutical composition comprising:
a nucleic acid; and a divalent cation, wherein
a total concentration of the divalent cation is 0.0406 mM or more and less than 81.1 mM, and
the liquid pharmaceutical composition is injected into an injection target by an injector configured to inject the liquid pharmaceutical composition into the injection target without using an injection needle.

2. The liquid pharmaceutical composition according to claim 1, wherein the divalent cation is one or more selected from the group consisting of Mg²⁺, Ca²⁺, Mn²⁺, and Zn²⁺.

3. The liquid pharmaceutical composition according to claim 1 or 2, wherein the injection into the injection target using the injector configured to inject the liquid pharmaceutical composition into the injection target without using an injection needle is injection into the injection target by jet injection.

4. The liquid pharmaceutical composition according to claim 1 or 2, wherein the liquid pharmaceutical composition is for inducing cellular immunity.

5. An injector configured to inject a liquid pharmaceutical composition into an injection target without using an injection needle, the injector comprising:
a storage section containing the liquid pharmaceutical composition;
a nozzle section communicating with the storage section, the nozzle section having an ejection port for ejecting the liquid pharmaceutical composition toward the injection target; and
a pressurization section for pressurizing the liquid pharmaceutical composition contained in the storage section during an operation, thereby ejecting the liquid pharmaceutical composition from the ejection port toward the injection target, wherein
the liquid pharmaceutical composition contains a nucleic acid and a divalent cation, and a total concentration of the divalent cation is 0.0406 mM or more and less than 81.1 mM.

6. The injector according to claim 5, wherein the divalent cation is one or more selected from the group consisting of Mg²⁺, Ca²⁺, Mn²⁺, and Zn²⁺.

7. A method for injecting a liquid pharmaceutical composition into an injection target, the liquid pharmaceutical composition containing a nucleic acid and a divalent cation and having a total concentration of the divalent cation of 0.0406 mM or more and less than 81.1 mM, the method comprising injecting the liquid pharmaceutical composition into the injection target using an injector configured to inject the liquid pharmaceutical composition into the injection target without using an injection needle.

8. The method according to claim 7, wherein the divalent cation is one or more selected from the group consisting of Mg²⁺, Ca²⁺, Mn²⁺, and Zn²⁺.
